(19) Europäisches Patentamt
European Patent Office
Office européen des brevets

(11) Publication number: 0 439 540 B1

(12) # EUROPEAN PATENT SPECIFICATION

(45) Date of publication of patent specification: 28.06.95

(51) Int. Cl.$^6$: A61K 39/395, C07K 16/00

(21) Application number: 89912460.6

(22) Date of filing: 23.10.89

(86) International application number:
PCT/GB89/01269

(87) International publication number:
WO 90/04413 (03.05.90 90/10)

(54) ANTIBODY CONJUGATES WITH TWO OR MORE COVALENTLY LINKED FC REGIONS.

(30) Priority: 24.10.88 GB 8824869

(43) Date of publication of application:
07.08.91 Bulletin 91/32

(45) Publication of the grant of the patent:
28.06.95 Bulletin 95/26

(84) Designated Contracting States:
AT BE CH DE FR GB IT LI LU NL SE

(56) References cited:
EP-A- 10 309
EP-A- 147 283
EP-A- 314 317
WO-A-89/01975
US-A- 3 928 580

CHEMICAL ABSTRACTS, vol. 110, no. 23, 05 June 1989, Columbus, OH (US); G.T. STEVENSON et al., p. 552, no. 210562a&NUM;

(73) Proprietor: 3i RESEARCH EXPLOITATION LIMITED
91 Waterloo Road
London SE1 8XP (GB)

(72) Inventor: STEVENSON, George, Telford
9 Meadowhead Road
Southampton SO1 7AD (GB)

(74) Representative: Sheard, Andrew Gregory et al
Kilburn & Strode
30, John Street
London WC1N 2DD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, vol. 107, no. 5, 03 August 1987, Columbus, OH (US); J. SIEGELMAN et al., pp. 524-525, no. 37765g&NUM;

CHEMICAL PHARMACEUTICAL BULLETIN, vol. 29, no. 4, 1981; T. KITAGAWA et al., pp. 1130-1135&NUM;

BIOCHEMICAL JOURNAL, vol. 101, 1966; J. STEPHEN et al., pp. 717-720&NUM;

NATURE, vol. 332, March 1988; L. RIECHMANN et al., pp. 326-327&NUM;

## Description

This invention relates to antibody derivatives having multiple Fc regions and intended to have improved utility where enhanced Fc activity is required for example in human cancer therapy or in the treatment of other conditions where the killing of mammalian cells is desirable.

Monoclonal antibody technology has made available large numbers of antibodies with specificities directed towards differentiation antigens on tumours. Among B-lymphocytic tumours - lymphomas and leukaemias - a variety of antigens typical of various stages of B-cell differentiation have been revealed. These supplement the well studied idiotype (Id) on surface immunoglobulin (Ig), a set of epitopes whose collective specificity can be regarded as essentially unique for individual tumours.

Unless the context requires otherwise, the terms "antibody" and "immunoglobulin" are hereinafter used synonymously.

It is evident that the use of antibody to ablate a neoplasm would be an acceptable means of therapy if any normal antigen-bearing tissue damaged simultaneously could either be regenerated from antigen-negative precursors or safely dispensed with altogether. By such a criterion the surface Id and a number of the other B-lymphocytic differentiation antigens are promising targets for antibody therapy. The major problem is that antibody, having apparently evolved to deal with microorganisms, is simply not a good killer of mammalian cells. Thus, to date the majority of patients with B-lymphocytic neoplasms who have been treated with anti-Id have shown only a partial and transient diminution in tumour load. Among the factors apparently enabling the target cells to escape are inadequate recruitment of effectors by the antibody, antigenic modulation, mutations affecting surface Id, and the development by the patient of an anti-antibody immune response.

One approach to rendering antibodies cytotoxically more effective has been the construction of chimeric derivatives, displaying antibody sites derived from xenogeneic antibody (usually mouse monoclonal IgG) and Fc regions derived from normal human IgG. The preparation of chimeric antibodies in which the antigen binding (Fab) arms of rodent IgG antibody are linked by thioether bonds to human IgG, or to the Fc gamma portion of human IgG, has been previously reported. Such a chimera has been prepared in quantity from monoclonal anti-Id and used in the treatment of human lymphoma [Hamblin et al., Blood, 42, 495 (1987)]. In this chemically derived chimera the advantages expected from displaying human instead of rodent Fc gamma are better recruitment of effectors (complement and various cells displaying Fc gamma receptors), a longer metabolic survival and a lower immunogenicity. A comprehensive review of work in this field is contained in Cancer Surveys Vol.4 No.1 (1985) (Stevenson and Glennie).

Recruitment of at least two important effector mechanisms to antibody-coated cells relies on interactions with modest intrinsic association constants, reinforced simply by multiple binding. The interaction between Clq heads and C-gamma-2, which initiates the classic complement pathway, has a $K_A$ estimated at only 1.8 to 5.8 x $10^4 M^{-1}$. To provide both firm binding and a conformational signal to the Clq, simultaneous engagement of two or more heads with neighbouring IgG molecules on the target cells is required. The interaction between the Fc-gamma-receptor type III (FcRIII) and Fc-gamma, necessary for the antibody-dependent cellular cytotoxicity (ADCC) effected by large granular lymphocytes, has a $K_A$ of approximately 1.1 x $10^7 M^{-1}$. The receptors are occupied efficiently only by entities capable of simultaneous multiple binding: thus lymphocytic ADCC can be inhibited by aggregated but not by monomeric IgG.

One object of the present invention is to provide antibody derivatives having improved Fc activity for example in regard to effector recruitment. Such derivatives are likely to have utility in the treatment of a range of conditions requiring the killing of mammalian cells for example in human cancer therapy, or in other conditions where enhancement of Fc function is beneficial.

According to the present invention there is provided a synthetic antibody derivative which comprises at least two Fc regions (as hereinafter defined) joined by covalent linkage and in which the number of any Fab regions (as hereinafter defined) present does not exceed the number of Fc regions.

Preferably the covalent linkage includes at least one synthetic chemical linking group.

Whilst the present invention should not be construed so as to include known natural structures, nonetheless, a range of structural forms may be obtained using the concept of the invention and a variety of preparative techniques may be employed in their manufacture.

Thus, the term "Fc region" as used herein and in the appended claims is intended to include not only, for example, typical immunoglobulin Fc structures comprising two peptide chains but also partial structures, active fragments and the like retaining Fc activity, e.g. recruitment effector ability or involvement in Ig transport and metabolism.

Fc regions may be derived from immunoglobulin of any class or species but if intended for human use are preferably from human immunoglobulin, e.g. IgG or IgG1. Polyclonal or monoclonal sources may be

used.

The term "synthetic" is intended to distinguish from naturally occurring structures such as IgM but should not otherwise be construed in a narrow sense.

The covalent linkage between Fc regions is preferably synthetic in character, but should not necessarily be construed so as to exclude naturally occurring types of bond which may have been broken and reformed or the possibility for example of forming certain structures by techniques of genetic engineering using recombinant DNA.

According to one aspect of the invention the covalent linkage includes a synthetic chemical linking group. The linkage may be direct, which term includes linkages containing a synthetic linking group, or indirect, by which is meant a link including an intervening moiety, e.g. one having antibody-type activity, such as an Fab region.

The antibody derivatives of the invention will usually have at least one Fab region but the inventive concept is not necessarily limited in this way. Multiple Fc regions may, for example, be useful per se as starting material for construction of more complex structures.

In any event the choice of Fab region is not fundamental to the inventive concept and may be made from any immunoglobulin which provides the desired specificity for target cells. The possibility is also envisaged of replacing complete Fab regions having typical structures comprising two peptide chains, with partial structures, active fragments and the like or alternative moieties having the desired antigen binding or receptor properties. For example, a lectin which specifically recognises carbohydrate moieties on a target cell may be used.

In general, therefore, the term Fab region as used herein and in the appended claims should be understood to include any entity having specifity for a target cell and any moiety having functional properties similar to an Fab region.

Fab regions are preferably derived from monoclonal antibodies although suitable polyclonals may also be used.

Suitable monoclonal antibodies include those reactive against human cells and which are of mouse isotype IgG1 or IgG2a. They should preferably be readily digested for example with pepsin, to yield fragments which on reduction generate Fab' gamma fragments containing the required sulphydryl groups.

In general herein, where Fc regions, Fab regions or synthetic chemical linking groups occur more than once in a structure or process it should be understood that they may be the same or different unless considerations of manufacture or use indicate otherwise.

According to further aspects of the present invention we provide:

$$Fab(A)\text{-}R\text{-}[\text{-}Fc\text{-}R]_m\text{-}Fab(B)$$

where,

Fab(A) is an Fab region from a first antibody A;

R represents a synthetic chemical linking group;

Fc represents an Fc region;

m is an integer greater than 1; and

Fab (B) is an optionally present Fab region from an antibody B which may be the same as, or different from A and which may be funtionally inert under conditions of use.

When Fab(B) is absent the linking group may for example be alkylated instead.

However, although the present invention provides the possibility of producing, in general, synthetic antibodies which have multiple Fc regions, a preferred antibody of the invention comprises two Fc regions derived from human Immunoglobulin G (IgG), most preferably IgG1.

In the above formula R preferably represents in each case a linking group linked via a thioether linkage to a cysteine residue in an Fab region or in an Fc region at one end and to a cysteine residue in an Fc region at the other end of the linking group. For reasons which appear below R is often the residue of a divalent linker molecule. However, in some cases it may be preferred that R shall be the residue of a trivalent linker molecule. It is alternatively possible for R, particularly in an -Fc-R-Fc- moiety, to represent a group of the formula:

-R'-X-R'-

where each R' represents a divalent linking group; and X represents a relatively bulky spacer molecule such as a protein or a peptide, for example plasma albumin.

An Fab region may contain up to about 11 cysteine residues in the hinge region. A typical Fab region has 3 cysteine residues in the hinge region. In the synthetic antibody of the invention two of these cysteine residues on an Fab region may be linked intramolecularly by a divalent group R whilst the third is linked by a divalent group R to an adjacent Fc region. If an Fab region is used that has only a single cysteine residue in the hinge region then again a divalent group R can be used to link the Fab region to an adjacent Fc region. However, if the Fab region has two cysteine residues only in the hinge region (e.g. an Fab region from mouse IgG3), then it may be expedient to use a trivalent group R to link the Fab region to an adjacent Fc region; in this case two of the valencies of the trivalent group R link to the two cysteine residues in the hinge region of the Fab region and the third to a cysteine residue on the Fc region.

The Fc regions are preferably linked one to another by a divalent group R. This can be the residue of a divalent linker molecule or it may be a group of the formula:

-R'-X-R'-

set out above.

A further representation of a synthetic antibody derivative, bisFabFc, of the invention is given in Figure 1 herewith, in which S is a sulphur atom.

A preferred example of a divalent linking group R is a divalent residue of a bis-maleimide. An example of a trivalent linking group R is the residue of tris-(2-chloroethyl)amine.

In the group -R'-X-R' -, the divalent group R' may be, for example, bound at one end by a thioether bond to a cysteine residue in the protein X and at its other end by a thioether bond to, for example, a cysteine residue of an Fc region.

The synthetic antibody of this aspect of the invention may be univalent with only one of its Fab arms directed against the target cell and the other Fab arm being of irrelevant antibody specificity, i.e. being functionally inert as regards the target cell. Alternatively, the synthetic antibody of the invention may be bivalent with both Fab arms having the same activity; or it may be bispecific with each Fab arm directed against a different epitope on the target cell.

The present invention also provides a method of preparing a synthetic antibody comprising the steps of:

(a) selectively reducing isolated $(Fab)_2$ fragments of an antibody to form Fab fragments containing one or more free sulphydryl groups in the hinge region thereof;

(b) derivatising the Fab regions by introduction of a first synthetic chemical linking group reactive with free sulphydryl groups to form a derivatised Fab region which can still be reacted with sulphydryl groups;

(c) reacting the derivatised Fab regions with a molar excess of Fc regions recovered from the same or from a different antibody and having a sulphydryl group in the hinge region, thereby forming a product (FabFc) having one Fc region, one Fab region attached to one Fc chain and a free sulphydryl group at the hinge region of the other Fc chain;

(d) subjecting the FabFc product to thiol-disulphide interchange;

(e) derivatising the thiol-disulphide interchanged FabFc product by introduction of a second synthetic chemical linking group; and

(f) linking the derivatised thiol-disulphide interchanged FabFc product with an FabFc product, prepared by the method of steps (a) to (d) above from the same or from a different antibody and having a sulphydryl group in the hinge region thereby to form bisFabFc in which the antigen binding arms Fab have the same or different specificities.

The separate Fab and Fc regions may be prepared by digestion of antibodies with pepsin or papain respectively and the selective reduction may be effected by treatment with dithiothreitol.

Derivatization may be effected by reaction of free suplhydryl groups with an at least difunctional linker molecule containing at least two functional groups capable of reacting with free sulphydryl groups. Examples of such functional groups include maleimido groups and chloro groups. Usually such a linker molecule contains two such functional groups; however, as explained above, it may be preferred to derivatise an Fab region with a trifunctional linker molecule, particularly when the Fab region has only two cysteine residues in the hinge region.

For the derivatization step (e) it is also envisaged that the thiol-disulphide interchanged FabFc product of step (e) may be linked to an inert protein, such as plasma albumin. If the inert protein does not already contain free sulphydryl groups these can be introduced by reaction with, for example, iminothiolane or 3-(2-pyridyldithio)propionic acid N-hydroxysuccinimide ester (SPDP). Linking of the sulphydryl groups on the inert protein with the thiol-disulphide exchanged FabFc product in step (e) can be effected by reaction with

a cross linker molecule.

Step (f) can then he carried out by linking of the inert protein to the second FabFc moiety by reaction of a linker molecule with free sulphydryl groups on the inert protein residue and on the second FabFc moiety.

It will often be preferred to effect derivatization in steps (b) and (e) in each case by reaction with a linker molecule having the desired number of functional groups (usually two functional groups) capable of reacting with free sulphydryl groups.

A particularly preferred type of linker molecule is a dimaleimide of the formula:

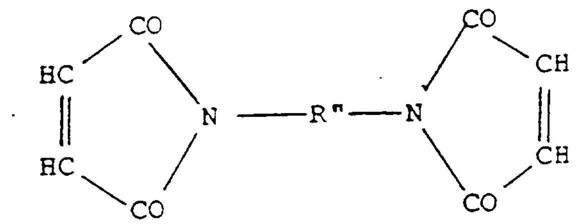

where R" is a divalent organic radical, for example an optionally substituted divalent aryl group or an optionally substituted divalent aliphatic group Such linker molecules include N,N'-o-phenylenedimaleimide and N,N'-bis(3-maleimidopropionyl)-2-hydroxy-1,3-propanediamine.

Other linker molecules which may be mentioned, particularly for linking certain types of Fab region to an adjacent Fc region, include tris-(2'-chloroethyl)amine.

The thiol-disulphide interchange may be effected by treatment with 2,2'-dipyridyldisulphide.

The sub-unit, FabFc, may under appropriate conditions, offer improvements over its parent antibody which are inherent in its chimerism: better effector recruitment, better metabolic survival and reduced immunogenicity. The subunits may be prepared in high yield from any monoclonal antibody which can be digested to F(ab' gamma)$_2$ fragments, a group which includes the common mouse isotypes IgG1 and IgG2a. The Fab Fc derivatives may then be used as such or dimerized in any combination of available specificities to yield the bisFabFc of the invention. The following further possible improvements in performance may be expected:

(i) If the derivative remains functionally univalent, that is with only one Fab arm directed against the target cell, the number of Fc arms per cellular epitope is doubled and the Fc pairing offers co-operative binding of effectors.

(ii) If the derivative is bivalent, there is increased affinity for the target cell together with Fc pairing.

(iii) In bispecific bisFabFc an increased affinity results from the simultaneous binding of its two target epitopes. Escape of the target cell due to its being at the low end of a constitutive distribution of antigen, or due to a mutation affecting the antigen, will be more difficult because two antigens must comply with these criteria simultaneously. Inactivation of the antibody by extracellular antigen is rendered more difficult, provided only one of the target epitopes is secreted. The advantage of Fc pairing is, of course, also manifest.

According to an alternative aspect of the present invention antibody derivatives may be represented by the formula:

Fc-R-Fab(-R-Fc)n

where;

Fc represents an Fc region;

R represents a synthetic chemical linking group;

Fab represents an Fab region;

n is an integer equal to or greater than 1; and

where the Fc's may be further extended by additional (-R-Fc) groups.

Such derivatives may also be represented as:

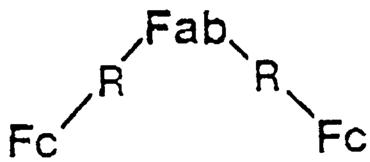


illustrating the indirect nature of the covalent linkage between the Fc groups which includes an Fab region.

The "parallel"-Fc geometry thus obtained offers the following potential advantages:

1. The parallel-Fc geometry is better suited to forming a regular array of Fc on the target cell for cooperative recruitment of effectors.

2. The preparation is simplified, and the yield of Fab in the final product improved.

3. The Fc hinges may be readily provided in either closed or open configuration (as defined below). In the latter form the derivatives will not activate complement, a feature which could prove advantageous when such activation is potentially toxic: in particular, when antibody is infused in the face of circulating soluble antigen.

By "closed" as used herein we mean that the peptide chains comprising the Fc region are linked by covalent bonding in or near the hinge region as illustrated in Figs. 6A to 6C of the accompanying drawings and described in more detail hereinafter.

The covalent bonding may conveniently terminate in cysteine residues in either chain and consist of disulphide links or thioether links incorporating a suitable synthetic chemical linking group.

By "open" we mean, conversely, that no covalent bonding of this kind is present.

More detailed description of methods for the preparation of antibody derivatives according to the invention and of structures so produced are given in the examples provided hereinafter.

The antibody derivatives of the invention are likely to be useful in a range of applications where enhanced Fc activity is required. They may form the active principle in pharmaceutical preparations in conjunction with pharmaceutically acceptable diluents or carriers as appropriate to the type of treatment or mode of delivery.

Although the derivatives are likely to be useful against a range of targets including microorganisms they are likely to be especially useful where the killing of mammalian cells is required for example in the treatment of conditions where cells display antigens which may be recognised by Fab regions of the derivatives of the invention.

Such projected applications include use against cancerous conditions, cells infected with a virus or other intra-cellular microbe or rogue auto-immune cells such as abnormal lymphocytic clones causing auto-immune disease.

Further possible applications include purging of unwanted cells from bone marrow and immunosuppression of graft recipients.

Thus for example it is envisaged that synthetic antibody derivatives with multiple Fc regions could be extended to include structures of the type described in this application, but in which the Fab has been replaced by another moiety with a sepcific affinity for a surface molecule on the selected target cell. For example, a target cell infected with human immunodeficiency virus, and displaying on its surface the viral protein gp120, could be targetted by $(CD4)Fc_2$ - a variant of $FabFc_2$ in which Fab has been replaced by the human normal protein for which the viral gp120 has a strong affinity. To make the product the CD4 would have to be provided with at least two sulphydryl groups near its C-terminus, by chemical or recombinant genetic means.

Derivatives provided by the present invention may therefore be useful in the treatment of AIDS.

Derivatives according to the invention may also have potential for targetting cells having Fc receptors in which case the Fab region would be used to bind the molecule which needs to reach the target, e.g. a toxin.

Derivatives may furthermore fund application in diagnostic tests and kits.

EXAMPLES

Examples of the invention will now be described with reference to the accompanying drawings in which:

Figure 1 is a diagramatic representation of a synthetic antibody derivative having a direct covalent linkage between Fc regions;

Figure 2 illustrates a method for the preparation of the derivative of Fig. 1;

Figure 3 is a graph of complement cytotoxity comparing derivatives according to the invention with another derivative;
Figure 4 shows graphs comparing the activity of a derivative according to the invention with other derivatives, 4A relating to complement cytotoxity and 4B to antibody-dependent cellular cytotoxity (ADCC);
Figure 5 is a diagramatic representation of a synthetic antibody having an indirect covalent linkage between Fc regions;
Figure 6 shows details of open and closed configurations for Fc regions;
Figure 7 illustrates a sub-routine used to produce an intermediate structure;
Figure 8 illustrates a part of an alternative sub-routine;
Figure 9 illustrates a method for the preparation of the derivative of Figure 5; and
Figure 10 is a graph of the killing of lymphoid cells by a derivative according to the invention compared with two other derivatives.

Antibody derivatives with direct covalent linkage

Referring to Fig. 1 the unshaded portions represent two Fc regions directly covalently linked to each other by a synthetic chemical linking group R terminating in the sulphur atoms of cysteine residues forming part of the chains of the Fc regions. The Fc regions have a closed configuration, the individual chains of each being covalently linked via disulphide links formed from cysteine residues in the chains.

Each Fc region is further covalently linked via similar -S-R-S- linkages to an Fab region (shown shaded in the drawing) derived from monoclonal antibodies A and B which may be the same or different. The complete synthetic antibody derivative is referred to by the nomenclature bisFabFc (A/B). Preparation and testing of derivatives as illustrated in Fig. 1 will now be described in more detail.

Materials

Dithiothreitol (DTT) from Sigma Chemical Company and 2-mercaptoethanol from British Drug Houses, were used without further purification. 2,2'-Dipyridyl disulphide (Py-SS-Py) from Aldrich Chemical Company was recrystallized from dimethylformamide (DMF)/water.

The bismaleimide crosslinkers, N,N'-o-phenylenedimaleimide (PDM) and N,N'-bis(3-maleimidopropionyl)-2-hydroxy-1,3-propanediamine (BMP) were each found upon titration to possess greater than 90% of the predicted maleimide activity and were used without further purification. Each was dissolved initially in DMF and, to minimise hydrolysis of the maleimide ring, diluted to the stated extent with aqueous buffer only immediately before use.

The affinity gel Protein A-Sepharose Cl-4B™ was obtained from Pharmacia. The cation-exchanger Phospho-Ultrogel A6R™ was obtained from Biotechnics (Villeneuve la Garenne, France).

Pepsin (from porcine gastric mucosa, product P6887, Sigma) was used without further purification. Papain was activated by incubation for 15 minutes at 37°C, pH 7.7 with 10mM 2-mercaptoethanol, 5mM EDTA. It was then passed at 20°C through Sephadex G-50™ (Pharmacia) equilibrated with 0.1M sodium acetate, pH 4.0, 1mM EDTA, 5.5mM 2-mercaptoethanol. The centre of the main protein peak was harvested and stored deep frozen in aliquots until required.

Preparation of Fc Fragments

Human normal IgG was prepared from plasma surplus to blood bank requirements. A set of basic plasma proteins were first prepared by binding to the cation-exchanger SP-Trisacryl™ (IBF Biotechnics) in 40mM Tris acetate, pH 6.0. After elution and buffer exchange they were led through DEAE-Sepharose FF™ (Pharmacia) in 50mM Tris HCl, pH 8.0, and the unretarded IgG which emerged was harvested. This procedure yielded only the basic half of the IgG population, these molecules having the advantage that their Fab and Fc fragments were readily separable by ion-exchange chromatography.

To prepare the Fc gamma fragments the IgG (18mg/ml) was digested at 37°C for 30 minutes with papain (0.05 mg/ml) in the presence of 0.5 mM 2-mercaptoethanol at pH 6.6. Digestion was stopped by adding N-ethylmaleimide (22 mM in DMF) to 2mM. The exposure to thiol entailed by these digestion conditions did not reduce any significant disulphide bonds in the IgG (less than 0.05/molecule by titration). The Fc gamma fragments were separated by sequential chromatography on Sephadex G-50™, DEAE-Sepharose FF™, and Sepharcryl S-200HR™ (all from Pharmacia). Passage through Sephadex G-50 separated IgG and its fragments from papain, and transferred them into 25mM Tris HCl, pH 8.0, for ion

exchange. On the DEAE-Sepharose column the Fc gamma and some of the undigested igG, but no Fab gamma, were retarded. The retarded material was eluted with 0.5 M sodium chloride, 0.1 M Tris HCl, pH 8.0 directly on to the Sephacryl for separation into IgG and Fc gamma. The Fc gamma fragments thus obtained were essentially entirely from IgG1: IgG2 is relatively resistant to cleavage under the digestion conditions used, IgG3 yields a large Fc gamma separated on the Sephacryl, and IgG4 is not present in significant amount in the basic IgG.

Preparation of Fab Fragments

Three mouse monoclonal antibodies were used. Anti-Id ($L_2C$), which is described in detail as anti-Id-1 by Elliott et al., Journal of Immunology, 138, 981 (1957) and by Stevenson et al. Br. J. Cancer (1984) 50, 407-413, is specific for the surface Ig idiotype on guinea pig, $L_2C$ leukaemic cells. Samples are available from Dr. M.J. Glennie of the Lymphoma-Research Unit, University of Southampton, Tenovus Research-Laboratory, Southampton General Hospital, Tremona Road, Southampton, SO9 4XY, UK. Anti-mu is specific for the heavy chains of human IgM and was raised in the laboratory and is available from Amersham International plc, Little Chalfont, HP7 9NA, UK. Anti-CD19, specific for the CD19 (p95) differentiation antigen on human B-lymphocytes, is secreted by the cell line RFB9 raised by, and available from, the Department of Immunology, Royal Free Hospital, Pond Street, London, NW3 2QG, UK.

F(ab' gamma)$_2$ from mouse IgG1 was prepared by limited digestion with papain. The progress of the digestion was monitored by chromatography of aliquots on Zorbax GF250™ (DuPont). With IgG1 at 18 mg/ml, pepsin 0.3 mg/ml and pH 4.1, the digestion was usually close to completion within 8 hours at 37°C, with the adjustment of pH being critical. F(ab' gamma)$_2$ was separated from the digest by recycling chromatography on Sephacryl S-200HR™, equilibrated with 0.5 M sodium chloride, 0.02 M Tris HCl and pH 8.0.

Preparation of FabFc and bisFabFc

Reference is made to Figure 2 herewith which summarises the steps involved in the preparation of FabFc and the bisFabFc.

F(ab'gamma)$_2$ from mouse IgG1 antibody is first reduced to yield Fab' gamma with free sulphydryl (SH) groups in its hinge region. These SH groups react with a surplus of bismalemide linker (PDM) to yield a free maleimide group attached to the hinge. The Fab' gamma (mal) now reacts with reduced human Fc-gamma-1 containing free sulphydryl in its hinge, the Fc being in molar surplus to discourage combination with more than one Fab'. The resulting FabFc is separated by gel chromatography and binding to Protein A. To facilitate its effector functions the Fc hinge is closed by using thiol-disulphide interchange to re-form the disulphide (SS) bond whose contributing cysteine residues have not been involved in the thioether link to Fab'. This leaves a lone cysteine available for further reaction with a linker (PDM or the longer BMP) to yield FabFc(mal) for union with Fabfc(SH). A variety of FabFc may be prepared, stored, used in this form, or, with further manipulation, dimerized by bisFabFc in any combination of specificities.

Selectively reduced Fab' gamma

The F(ab' gamma)$_2$ fragment obtained by peptic digestion of mouse IgG antibody was reduced with DTT at pH 8.0, allowed to undergo thiol-disulphide interchance with 2,2'-dipyridyldisulphide at pH 5.0 and again reduced with DTT at pH 5.0. After passage through Sephades G-25™ the resulting protein was found to have 2.9 titratable SH groups per kD, to migrate as 94% monomer, 6% dimer on gel filtration in Sephacryl S200™ and to undergo little separation into its Fd' gamma and light chain consituents on SDS-PAGE. The predominant species (greater than 90%.) was therefore monomeric Fab' gamma with its gamma-light SS bond intact, but with three SH groups derived from reduced inter-gamma chain bonds.

Derivatization of Fab'-gamma

After being passed through Sephadex G-25™ in 20mM sodium acetate, 1mM EDTA, pH 5.3, Fab'-gamma (SH) was allowed to react with 2mM at this pH, at 5°C for 30 minutes. DMF was present at 20% v/v. At the end of incubation one fifth volume of cold acetic acid was added, bringing the pH to 4.6. To remove the PDM and concentrate the protein, the solution was passed through Phospho-Ultrogel A6R™, in 10% DMF/90% $Na_2EDTA$, at 5°C. After rinsing, the Fab'-gamma(mal) was reverse-eluted with 0.5M sodium acetate, pH 5.3, and collected in an ice bath under nitrogen. Phospho-Ultrogel™ has the advantage of

permitting compact elution at pH 5.3, so avoiding an increased rate of hydrolysis of maleimide groups at the higher pH values found necessary with other cation-exchangers.

Protein was collected at 4 to 8 mg/ml. Aliquots chromatographed on Sephacryl S-200HR™ revealed a dimer content of less than 5%, with higher aggregates usually not discernible. In four assays the maleimide content varied between 0.84 and 1.05 per molecule, sufficiently close to 1.0 in view of the approximations involved. No residual SH was detectable with Py-SS-Py. These findings imply that two of the hinge region SH groups have cyclized with the linker, leaving the third to react with one end only. Such a result is very useful, as the presence of more than one maleimide group attached to the Fab' hinge will increase the tendency to form higher order aggregates when Fab' reacts with human Fc-gamma.

The longer linker BMP has the advantage of greater solubility than PDM, and a greater stability of its malemide groups due to their being alkyl- rather than aryl-linked. However, when used in the above protocol at 2 mM it gave a greater tendency to cross-link the Fab', giving a dimer content of greater than 15%. When its concentration was raised to 4mM the amount of dimer formed was less but it then yielded 1.3 to 1.6 maleimide units per Fab', and was associated with greater formation of aggregates in the Fab'-Fc interaction than was seen using PDM. Both of these problems with BMP are explicable on the basis of its being less likely than PDM to cross-link intramolecularly two of the three available SH groups.

Formation of FabFc

Human Fc-gamma was reduced by reaction with 6 mM DTT, pH 8.0, for 15 minutes at 37 °C, passed through Sephadex G-25 in 0.5 M sodium acetate, pH 5.3, and collected in an ice bath under nitrogen. Titrated SH groups have been in the range 3.2 to 3.6 per molecule, to be compared with a theoretical 4.0. Examination on SDS-PAGE revealed no surviving protein in the 50 kD postion, and hence no surviving hinge SS group. The relatively low titration suggest some proteolytic loss of hinge.

Immediately upon its being prepared Fab'-gamma(mal) was added to constantly stirring Fc(SH) at a molar ratio Fab:Fc of 1:2.5. The mixture, with a minimum protein concentration of 7 mg/ml, was incubated at room temperature for two hours. Any reduction in the molar excess of Fc increased the amount of aggregate which formed, the aggregate being interpreted as mainly greater than one Fab per Fc. Economically a waste of Fc is much less serious than a waste of the antibody Fab, and the excess Fc is not usually recovered.

FabFc was separated from aggregate and from surplus Fc by recycling chromatography on Sephacryl S200HR™ in 0.5 M sodium acetate, 5mM EDTA, pH 5.8, at 5 °C. 2-mercaptoethanol was present at 2mM to inhibit a small amount of oxidative dimerization of Fc which otherwise tended to occur. Typically the separated aggregate amounted to about 10% of the protein present in the FabFc (100 kD) peak. The FabFc was led from the Sephacryl™ on to a Protein A-Sepharose™ column to which it, but not any contaminating F(ab'gamma)$_2$, binds.

Thiol-disulphide interchange to close the Fc hinge was carried out at pH 5.3 while the FabFc remained bound to the Protein A column. After rinsing off the 2-mercaptoethanol a solution of 0.1 mM Py-SS-Py in 5% DMF was passed through the column at 5 °C for 30 minutes. There was a prompt release of 2-thiopryridone, which appeared at the front of the emerging Py-SS-Py. The amount released, when related to the amount of protein subsequently eluted, indicated a reaction with 2.3 to 2.8 SH groups per FabFc (theoretical 3.0).

At this stage, with its unlinked hinge cysteine present as a 2-pyridyldisuplhide, the FabFc may be eluted for immediate use or for a subsequent incorporation into bisFabFc. If it is proposed to proceed at once to bisFabFc, the protein may be left on the column for further manipulation.

The recovery of Fab' in FabFc, in terms of F(ab'-gamma)$_2$ used at the outset, is about 65%.

Formation of bisFabFc

Two lots of FabFc, of the sane or different antibody specificities, were isolated on separate Protein A columns. On one the unlinked cysteine was returned to the SH form, on the other it was gven a maleimide group by reaction wth a bismaleimide linker. The linker was usually BMP, chosen because its long alkyl chain should permit greater separation of the two Fc than would PDM, thereby faclitating the simultaneous binding of effector molecules to each Fc. However, a comparison of the two linkers in the inter-Fc postion failed to demonstrate any clear advantage in effector recruitment. Therefore, BMP was generally used because of its greater solubility. Amounts of FabFc were adjusted to give a molar ratio of FabFc(SH):FabFc-(mal) of 1:1.1, to allow for some attrition of the maleimide.

In detail, FabFc on both Protein A columns had their unlinked hinge cysteine (present as a 2-pyridyldisulphide) selectively reduced by passage of 0.7 mM DTT, pH 5.3, through the columns at 5°C for 30 minutes. The effluent DTT was accompanied by 2-thiopyridone, 0.80 to 0.92 molecule per FabFc. This figure confirms that the earlier reaction with Py-SS-Py must have largely re-established the SS bond between the paired cysteines in the Fc hinge. To prepare FabFc(mal) one of the preparations was allowed to react with 8mM BMP in 12.5% DMF, 20 mM sodium acetate, pH 5.3, passed through the column at 5°C for 30 minutes.

After washing surplus reagents from the columns, FabFc(SH) and FabFc(mal) were eluted in the one solution by passing 0.1 M glycine hydrochloride through the columns in tandem. The pH was adjusted to 5.5 with sodium acetate and the solution incubated at 20°C for 16 hours. The elution volume was kept to a minimum by attention to column geometry: a protein concentration of greater than 10 mg/ml is desirable because the formation of thioether bonds competes with hydrolysis of maleimide.

At the end of the incubation period, chromatography of aliquots on Sephacryl S200™ revealed that up to 77% of the protein migrated as a 200kD species. Apart from a minute amount of higher aggregate, the remainder behaved as 100 kD species. Examination on Ouchterlony plates revealed that the 200 kD species contained mouse Fab and human Fc epitopes on the one molecule, and revealed no molecules containing epitopes of only one type. this fraction is therefore regarded as essentially entirely bisFabFc. The 100 kD fraction contained molecules with both the Fab and Fc epitopes, i.e. FabFc, as its principal constituent, but there was a small amount of putative $Fc_2$.

The entire preparation may be separated chromatographically into its bisFabFc and FabFc components if so desired. However, for therapeutic use there is no need to do so as a small amount of FabFc is not obviously disadvantageous. Additional steps required for therapeutic preparations are the removal of pyrogens, by meticulous exclusion throughout or by partial exclusion followed by chromatography on a polymyxin column; and passage through a bacterial filter (0.22 micrometres) imediately before collection into a sterile container.

Effector Functions of bisFabFc

Three preparations with the following specifications were examined: (a) anti-Id/anti-Id, with both arms directed against surface Id on guinea-pig $L_2C$ cells; (b) anti-Id/0 with one anti-Id ($L_2C$) and one irrelevant arm (from an IgC1 anti-Id raised against a human tumour), effectively univalent for $L_2C$ cells; (c) anti-mu/anti-CD19, directed against the indicated antigens on human lymphoid cells. Preparations (a) and (b) were tested against $L_2C$ cells, preparation (c) against the human B-lymphoblastoid line Daudi. None of the parent mouse IgG1 antibodies showed any activity whatever in assays of complement lysis or lymphocytic ADCC against these targets.

Figure 3 shows that for complement lysis bisFabFc (anti-Id/0) had about twice the titre of the parent FabFc when considered by weight, and about four times the titre as judged by molarity of active antibody sites. Two factors could have contributed to this superiority, the increased number of Fc on the cell and the juxtaposition of Fc in pairs. These factors cannot be separately evaluated at present.

BisFabFc (anti-Id/anti-Id) outperformed the univalent bis derivative whether charted by weight (Figure 3) or by molarity of active antibody sites. A contribution to this improvement must have arisen from the increased affinity associated with bivalent binding, but a negative factor could have been a greater tendency for the bivalent derivative to induce antigenic modulations of the surface Ig [Gordon & Stevenson, Immunology, 42, 13 (1981)]. In fact flow cytofluorimetry did not reveal any modulation of $L_2C$ cells induced by bisFabFc (anti-Id/anti-Id) over a period of 30 minutes: this may relate to the long flexible inter-FabFc link, or to the fact that the parent IgG1 anti-Id is itself a poor modulator with a probable "monogamous" mode of binding to the surface Ig [Elliot et al, Journal of Immunology 138, 981 (1987)].

In Figure 4 the performance of bisFabFc (anti-mu/anti-CD19) is compared with those of both parental FabFc. In ADCC the bis derivative revealed a titre about 16 times that of either parent. The complement killing was poor overall but the superiority of the bis derivative is clear. Again these simple assessments do not permit the contributions of multiple factors - binding affinity, Fc per cell, juxtaposition of Fc, and possible incipient modulation - to be disentangled.

On no occasion have assays of complement lysis revealed a prozone which would suggest anti-complementary activity of bisFabFc.

Antibody-invoked Cytotoxicity

Complement-mediated killing of $^{51}Cr$-labelled, antibody-coated cells was carried out by standard methods. The complement source was fresh serum from normal rabbit or guinea pig, at a final dilution of 1:5. ADCC was carried out using freshly isolated lymphocytic effectors from normal human blood, at an effector:target cell ratio of 50:1. Human AB serum (inactivated) was present at 10%. The extent of lysis was read at 3.5 hours.

Percentage cytotoxicities represent the means of duplicates, calculated in the standard manner, that is:

$$\frac{\text{counts released with antibody} - \text{counts released with normal IgG}}{\text{counts released with detergent} - \text{counts released with normal IgG.}}$$

Antibody derivatives with indirect covalent linkage

Referring now to Fig. 5 of the drawings the unshaded portions represent two Fc regions indirectly linked to each other via a covalent linkage which includes an intervening Fab region (shaded). Links between regions involve synthetic chemical linking groups R terminating in sulphur atoms of cysteine residues in the individual chains of the regions. As shown, both chains of the Fab regions are linked to an Fc region but it will be appreciated that, provided enough reactive groups are present, both Fc regions could be linked to one chain. The complete derivative is referred to by the nomenclature Fab $Fc_2$.

More details of Fc regions with closed or open configurations are shown in Figure 6 in which 6A-6C are closed and 6D is open.

The hinge in $FabFc_2$ (closed) is closed by reconstruction of one of its disulphide bonds, or by tandem thioether bonds separated by the group R', in homologous (Figure 6A) or non-homologous (Fig 6B) linkage.

S● indicates a thiol group rendered inert by alkylation or other means.

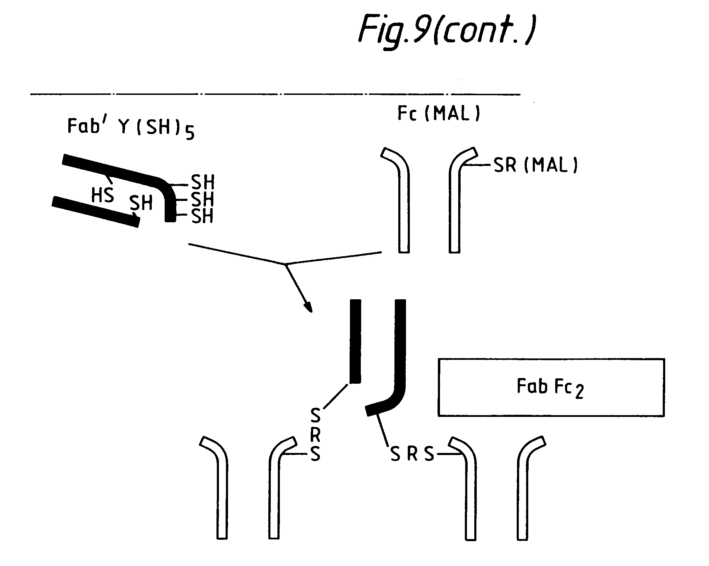

A random element in the chemical synthesis means that possibly any two of the five indicated S atoms on Fab can participate in the linkages to Fc; and in turn that any one of the four S atoms in the Fc hinge can be involved in a linkage to Fab. (See the fuller structural formulae in Fig. 9).

Details of methods for the preparation of antibody derivatives of the type illustrated in Fig. 5 now follow.

Principles

1. The basic preparative step involves the reaction of Fc gamma having a single maleimide group in its hinge, Fc (mal), with Fab which has free SH groups (Fig. 9). The SH groups in Fab arise from reduction of all its interchain disulphide bonds, and in the majority of cases (Fab' derived from mouse monoclonal IgC1 or IgG2a) such Fab will have five SH groups: Fab $(SH)_5$. In other cases the number of groups could vary from three (e.g. from mouse IgG3) to six (e.g. from mouse IgG2b), but such variation will entail no more than minor amendments to our preparative procedure.
2. It is found that the reaction of Fc(mal) with $Fab(SH)_5$, in a molar ratio of Fc:Fab $\geqq 3$, yields $FabFc_2$ as its major product (Fig. 9).
3. The minor products are $FabFc_3$ and FabFc: these can be removed, but present indications are that no harm accrues from leaving them with the $FabFc_2$ in order to improve the yield of product.
4. The reason for $FabFc_2$ being the major product, even when the reaction mixture contains a molar ratio of Fc:Fab $\geqq 3$, is that steric hindrance discourages further access of Fc(mal) to SH groups after the formation of $FabFc_2$.
5. The preparation of Fc(mal) entails a subroutine which leaves the Fc gamma as an intermediate product Fab-SS-Fc (sacrificial FabFc) for storage. The Fab moiety (at present sheep Fab' gamma) is discarded, although some recycling is possible. The subroutine makes Fc available in a univalent combining form: this simplifies the products of the final (Fab + Fc) reaction, preventing in particular the formation of species with more than one Fab.
6. In sacrificial FabFc the sheep Fab' gamma blocks a single cysteine residue in the Fc hinge. This permits the substitution of a single maleimide group on the Fc in one of two ways.

a. Subroutine (1), used for the preparation of both Fc(mal) with open hinges and Fc(mal) with thioether-bonded hinges. The cysteine residue on which the sheep Fab' gamma is placed is that which eventually bears the maleimide group.

b. Subroutine (2), used for preparation of Fc(mal) with disuphide-bonded hinges. The cysteine residue which bears the maleimide group is directly opposite the residue bearing the sheep Fab' gamma.

Subroutine (1) for sacrificial FabFc (Fig. 7)

1. The F(ab' gamma)$_2$ fragment obtained by peptic digestion of sheep normal polyclonal IgG is reduced with dithiothreitol (DTT) at pH 8.0 and allowed to undergo thiol-disulphide interchange with 4,4'-dipyridyldisulphide at pH 5.0, as described previously for mouse Fab' gamma (Principles para 1). The product (Fab-SS-Py) has a single mixed pyridyl disulphide at the site of the former inter-Fab disulphide bond.

2. The interchain disulphide bonds of human Fc gamma are reduced by reaction with DTT as described previously (Principles para 1).

3. Fab-SS-Py and Fc(SH)$_4$ are allowed to undergo disulphide interchange at 25 °C and pH 4.4, with Fab and Fc equimolar. At this pH pyridyldisulphides are uniquely susceptible to the nucleophilic attack by thiol which is entailed in disulphide interchange. In contrast the disulphide bond which is formed between Fab and Fc undergoes little degradation at the acid pH, despite the proximity of protein SH groups (Fig. 7), before the SH groups are rendered inert by alkylation. The disulphide-bonded products which form are FabFc (major) and Fab$_2$Fc (minor), with very little larger. With the total protein concentration at 5 mg/ml or greater, 30 minutes is sufficient for this reaction.

4. If the Fc hinge is required in open form, disulphide interchange is stopped by alkylation with N-ethylmaleimide at pH 5.0.

5. If the Fc hinge is required in closed form the alkylation is carried out with the bivalent linker o-phenylenedimaleimide (Fig. 7). The linker can bridge the three remaining SH groups in three ways, two of which result in the hinge being closed. Consistent with this reasoning the majority of Fab-SS-Fc molecules so alkylated are found by electrophoresis in a dissociating medium to have the halves of their Fc components covalently joined. The unbridged SH group will react with the linker to leave a free maleimide group, which can be inactivated by brief exposure to 2-mercaptoethanol at pH 5.0.

6. Sacrificial FabFc is separated from other reaction products and from residual reactants by gel chromatography on Sephacryl S200HR™ (Pharmacia).

7. The sacrificial FabFc is stored at 5 °C until required, and has proved stable under these conditions for at least three months.

Subroutine (2) for sacrificial FabFc (Fig. 8)

1. )
2. ) As for Subroutine (1) immediately above.
3. )

4. Dipyridyldisulphide is added to 0.2 mM and disulphide interchange allowed to proceed for a further 30 minutes at 25 °C. As a result those hinge disulphides where both cysteine residues remain available are closed (see reference (1) for a discussion of the mechanism of closure of protein disulphide bonds by such interchange), and other residual cysteine residues are converted to the mixed disulphide protein-SS-Py.

5. Sacrificial FabFc, now in a stable form with the disulphide configuration Fab-SS-Fc-SS-Py (Fig. 8), is separated by gel chromatography on Sephacryl S200HR™, and stored at 5 °C until required.

Main Reaction

The major features of the main reaction, joining mouse Fab' gamma to human Fc, are set out in Fig. 9. There are some differences in detail according to whether the sacrificial FabFc used comes from Subroutine (1) or Subroutine (2).

Referring to Fig. 9, only those bonds joining the mouse Fab' gamma and human Fc moieties are shown, so that the diagram suffices for all variants of FabFc$_2$: with open hinges, thioether-bonded hinges, and disulphide-bonded hinges. In the preparation of the last of these variants sheep Fab' gamma remains attached to the Fc, opposite the maleimide group, until after the Fc has been linked to antibody Fab' gamma

A. Using sacrificial FabFc from Subroutine (1)

1. F(ab' gamma)$_2$, derived from peptic digestion of mouse monoclonal IgG antibody, is reduced with 3 mM DTT at 25°C and pH 8.0 to yield Fab(SH)$_5$. The pH is then lowered to 5.0 and the ionic strength to 0.02 or less (by addition of dilute acid, or by passage through Sephadex G-25™ (Pharmacia) equilibrated with 0.02 M acetate buffer of pH 5.0), permitting the protein to be led onto and bound to the cation-exchanger Phospho-Ultrogel 6B™ (I.B.F. Biotechnics).

2. Sacrificial FabFc is led onto a column of protein A-agarose, which has a high affinity for Fc gamma. A solution of 6mM DTT in buffer at pH 8.0 is put through the column at 5°C, reducing the inter-subunit disulphide bond and releasing the sheep Fab' gamma in the column effluent. This Fab' gamma may be collected for re-use if desired.

3. Fc(SH) is now eluted from the column with 0.1 M glycine-HCl, pH 3.0, and allowed to react with a bis-maleimide linker. Linkers which have been used are N,N'-o-phenylenedimaleimide at 2 mM and N,N'-bis-(3-maleimidopropionyl)-2-hydroxy-1,3-propanediamine at 5 mM. The linkers are dissolved in dimethylformamide prior to being added to the protein solution. Final conditions for attachment of the linker are Fc 0.25 mM, linker at 2 or 5 mM, dimethylformamide up to 20% V/V, pH 5.3, 5°C. After 30 minutes the protein, now in the form of Fc(mal), is separated from the linker and transferred into 0.5 M acetate buffer, pH 5.3, by passage through Sephadex G-25™ (Pharmacia) at 5°C.

Longer and more rigid linkers, e.g. peptides with two attached maleimide groups, could be contemplated.

4. Upon emerging from the Sephadex column the Fc(mal)-containing solution is led onto the Phospho-Ultrogel 6B™ column, thereby eluting the bound antibody Fab (SH)$_5$ and yielding both proteins in a single concentrated solution (total protein about 10mg/ml). The molar ratio Fc:Fab is three.

5. The reaction Fab(SH)$_5$ + Fc(mal) is allowed to proceed at pH 5.3 and 20°C for 16 hours. Residual SH groups are then removed by alkylation with N-ethyl-maleimide. Finally the products are separated, on the basis of size differences, by recycling chromatography through Sephacryl S-200HR™ (Pharmacia).

B. Using sacrificial FabFc from Subroutine (2)

The approach here is to prepare Fc(mal) and link it to antibody Fab(SH)$_5$ before removing the sheep Fab' gamma from the Fc. Steps 2 and 3 describe reduction and maleimidation at the Fc cysteine residue opposite that bonded to sheep Fab' gamma, keeping the pH low (4.O) so as to avoid reducing either the disulphide bond by which the sheep Fab' gamma is attached or the disulphide bond in the hinge.

1. As in A.

2. Sacrificial FabFc, in the form Fab-SS-Fc-SS-Py, is allowed to react with dithiothreitol (O.5 mM) at pH 4.0 and 5°, for one hour. It is thereby converted to Fab-SS-Fc-SH, which is separated on Sephadex G-25™ equilibrated with pH 4.0 buffer.

3. The FabFc now reacts with 3nm o-phenylenedimaleimide in 0.1 M acetate, pH 4.0, 25% v/v dimethylformamide, at 5°C for two hours. It is thereby converted to Fab-SS-Fc(mal) which is separated and concentrated by binding to a column of Phospho-Ultrogel 6B™.

4. The two Phospho-Ultrogel™ columns (from stages 1 and 3) are eluted in series with 0.5 M acetate, pH 5.3. The Fab-SS-Fc(mal) and Fab(SH)$_5$, at a molar ratio of 3:1, are allowed to react in concentrated solution (total protein about 12 mg/ml) for 16 hours at 25°C. The principal product is (Fab-SS-Fc)$_2$Fab.

5. The pH is raised to 6.0 and the proteins led through a column of Protein A-agarose, thereby binding all molecular species containing a human Fc component. Sheep Fab' gamma is now released from combination by passing 2 mM mercaptoethanol through the column at pH 6.0 for 30 minutes. Finally, to encourage re-formation of any hinge disulphide which may have been reduced, and to block the cysteine SH group left after release of the sheep Fab' gamma, 2 mM cystamine is passed through the column at pH 7.0 for 30 minutes.

6. Finally FabFc$_2$ and other products are separated, on the basis of size differences, by recycling chromatography through Sephacryl S-200HR™ (Pharmacia).

References

1. G.T. Stevenson, A. Pindar, C.J. Slade. A chimeric antibody with dual Fc regions (bisFabFc) prepared by manipulations at the IgG hinge. Anti-Cancer Drug Design 3: 219-230, 1989.

2. K. Brocklehurst. Specific covalent modification of thiols: applications in the study of enzymes and other biomolecules. Internat. J. Biochem. 10: 259-274, 1979.

FUNCTION

Preliminary assessments of function of $FabFc_2$ with parallel Fc moieties are illustrated with reference to Fig. 10 which is a graph plotting the killing of human lymphoid cells (Daudi-line, labelled with radioactive chromium) by three univalent chimeric antibody derivatives (specificity, anti-CD19) in the presence of human effectors (normal blood lymphocytes), effector: target = 50). Cytotoxicity was measured after 3.5 hours at 37°C. A comparison is made of $FabFc_2$ (open hinge) with FabFc (open hinge) and FabFc (closed hinge), in this short-term test of antibody-dependent cellular cytotoxicity (ADCC), which is probably the best of those available in vitro for predicting the capacity of an antibody to kill target cells in vivo. The $FabFc_2$ is seen to be active in the test, and superior to its analogues containing only one Fc component.

Antibody derivatives have also been made in which the Fab region is derived from a monoclonal antibody having the following specifications.

| | |
|---|---|
| Clone: | WR17 (Obtainable from the Wessex Regional Immunology Service, Southampton, UK) |
| Isotype: | Murine IgG2a |
| Fusion details: | Conventional Kohler-Milstein type fusion using spleen cells from a Balb/c mouse immunised with cells from a patient with leukaemia and the mouse myeloma line NS-O. |
| Specificity: | WR17 is an anti-CD37 reagent, reacting with a 40-52kDa glycoprotein expressed strongly by B lymphocytes except in their early and late stages of development. This antigen is also weekly expressed by granulocytes and macrophages, and occasionally by T-cell lymphomas. |
| Applications: | WR17 can be used as a primary antibody to detect CD37 +ve cells in suspensions and in frozen sections. |

Preliminary indications of activity in an $FabFc_2$ derivative of this type were.

1. The $FabFc_2$ was seen by cytofluorimetry to bind to human B lymphocytes prepared from normal blood, and to the B-lymphocytic lines Namalwa and Daudi.
2. The $FabFc_2$ rendered Daudi cells susceptible to lysis by xenogeneic (guinea pig) complement, with 50% of plateau killing occurring at an antibody concentration of 1.6 micrograms/ml.
3. The $FabFc_2$ also rendered Daudi cells susceptible to ADCC by human blood lymphocytes, with 50% of plateau killing occurring at an antibody concentration of 50 nanograms/ml.

Further tests were also performed with an $FabFc_2$ derivative having its Fc components in serial configuration and comparing activity with an FabFc derivative. Mouse Fab' gamma and human Fc were used in each case. Experimental details were as follows:

The test concerned antibody therapy of a transplantable B-lymphocytic leukaemia ($L_2C$) of guinea pigs. $10^5$ cells were inoculated intraperitoneally into three groups of 8 animals on day 0. Treatment of two groups consisted of a single does of 0.2mg antibody derivative, of anti-idiotype specificity, injected intraperitoneally at 24 hours. The control group of animals received phosphate-buffered saline (PBS). The doubling time of the tumour is approximately 19 hours, so on a simple kinetic basis each halving of tumour load prolongs life by a further 19 hours.

A graph of animals surviving plotted against days after inoculation showed the following:

1. PBS. A steep decline after 12 days to no survivors after 14 days.
2. FabFc. A slightly less steep decline after 15 days to no survivors after 20 days.
3. $FabFc_2$. A gradual decline after 15 days to 6 survivors after 20 days followed by a steep decline to one survivor after 21 days still surviving after 24 days.

## Claims

**1.** A synthetic antibody derivative which comprises at least two Fc regions joined by covalent linkage (wherein the term "Fc region" includes both immunoglobulin Fc structures and active fragments thereof and partial structures), characterised in that the number of Fab regions present does not exceed the number of Fc regions (wherein the term "Fab region" includes any entity having specificity for a target cell and any moiety having functional properties similar to an Fab region).

**2.** An antibody derivative as claimed in claim 1 characterised in that the covalent linkage includes at least one synthetic chemical linking group.

3. An antibody derivative as claimed in claim 1 or claim 2 characterised in that, at least part of the covalent linkage between Fc regions is essentially direct.

4. An antibody derivative as claimed in any one of the preceding claims characterised in that it comprises at least one Fab region.

5. An antibody derivative as claimed in any one of the preceding claims characterised in that it is represented by the formula:

Fab(A)-R-[-Fc-R-]$_m$-Fab(B)

where,
Fab(A) is an Fab region from a first antibody A;
R represents a synthetic chemical linking group;
Fc represents an Fc region;
m is an integer greater than 1; and
Fab (B) is an optionally present Fab region from an antibody B which may be the same as, or different from A and which may be functionally inert under conditions of use.

6. An antibody derivative as claimed in claim 5 characterised in that;
Fab(A) is an Fab region from a mouse monoclonal antibody specific for the heavy chains of human IgM, referred to hereinbefore as anti-mu;
R is derived from a bismaleimide linker group;
Fc is an Fc region derived from human immunoglobulin IgG;
m equals 2; and
Fab(B) is an Fab region from a mouse monoclonal antibody specific for the CD19 differentiation antigen on human B-lymphocytes, referred to hereinbefore as anti-CD19.

7. An antibody derivative as claimed in any one of claims 1, 2 or 4 to 6 characterised in that at least part of the covalent linkage is indirect in that it includes an Fab region.

8. An antibody derivative as claimed in claim 7 characterised in that it is represented by the formula:

Fc-R-Fab(-R-Fc)n

where;
Fc represents an Fc region;
R represents a synthetic chemical linking group;
Fab represents an Fab region;
n is an integer equal to or greater than 1; and
where the Fc's may be further extended by additional (-R-Fc) groups.

9. An antibody derivative as claimed in claim 8 characterised in that;
Fc is an Fc region derived from human immunoglobulin IgG;
R is derived from a bismaleimide linker group;
Fab is an Fab region from a mouse monoclonal antibody specific for the CD37 differentiation antigen on human B-lymphocytes; and
n equals 1.

10. An antibody derivative as claimed in any one of the preceding claims characterised in that it has at least one Fc region with an open hinge configuration.

11. An antibody derivative as claimed in any one of the preceding claims characterised in that it has at least one Fc region with a closed hinge configuration.

12. An antibody derivative as claimed in claim 11 characterised in that
the configuration is closed via bonding including a synthetic chemical linking group.

**13.** An antibody derivative as claimed in claim 11 characterised in that the configuration is closed via a disulphide bond terminating at each end at a cysteine residue.

**14.** An antibody derivative as claimed in any one of claims 10 to 13 characterised in that free sulphydryl groups on the Fc regions have been rendered inert for example by alkylation.

**15.** An antibody derivative as claimed in any one of the preceding claims characterised in that it includes at least one link between an Fab region and an Fc region the said link comprising a synthetic chemical linking group.

**16.** An antibody derivative as claimed in any one of the preceding claims characterised in that at least one of the Fc regions is derived from human immunoglobulin for example IgG or IgG1.

**17.** An antibody derivative as claimed in any one of the preceding claims characterised in that it has an Fab region at least part of which is derived from non-human immunoglobin.

**18.** An antibody derivative as claimed in any one of the preceding claims characterised in that it has at least one Fab region derived from monoclonal antibody.

**19.** An antibody derivative as claimed in any one of the preceding claims characterised in that it has at least one synthetic chemical linking group linked via a thioether linkage to a cysteine residue in an Fab region or an Fc region at one end and to a cysteine residue in an Fc region at the other end of the linking group.

**20.** At antibody derivative as claimed in any one of the preceding claims characterised in that it has at least one synthetic chemical linking group which comprises a residue of a divalent or trivalent linker molecule.

**21.** An antibody derivative as claimed in claim 20 characterised in that the residue is a divalent residue of a bis-maleimide.

**22.** An antibody derivative as claimed in claim 21 characterised in that the bis-maleimide is a dimaleimide of the formula:

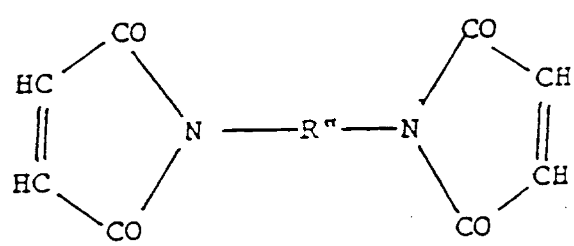

where R" is a divalent organic radical, for example an optionally substituted aryl or alkyl group.

**23.** An antibody derivative as claimed in claim 20 characterised in that the residue is a residue of a trivalent linker molecule linking an Fab region having two cysteine residues with an Fc region.

**24.** An antibody derivative as claimed in claim 23 characterised in that the residue is a residue of tris-(2-chloroethyl) amine.

**25.** An antibody derivative as claimed in any one of the preceding claims characterised in that it has at least one synthetic chemical linking group which includes a relatively bulky spacer molecule to increase the distance separating those parts of the antibody which are linked thereby.

**26.** An antibody derivative as claimed in claim 25 characterised in that the spacer molecule is derived from an inert protein, e.g. plasma albumin.

**27.** A pharmaceutical preparation characterised in that it comprises as an active principle an antibody derivative as provided by any one of the preceding claims with a pharmaceutically acceptable diluent or carrier.

**28.** The use of an antibody derivative as claimed in any one of claims 1 to 26 for the preparation of an agent for the treatment or prevention of cancerous conditions.

**29.** The use of an antibody derivative as claimed in any one of claims 1 to 26 for the preparation of an agent for killing mammalian cells, for example, cells infected with a virus or other intra-cellular microbe, or rogue auto-immune cells such as abnormal lymphocytic clones causing auto-immune disease.

**30.** The use of an antibody derivative as claimed in any one of claims 1 to 26 for the preparation of an agent for purging unwanted cells from bone marrow.

**31.** The use of an antibody derivative as claimed in any one of claims 1 to 26 for the preparation of an agent for the immunosuppression of graft recipients.

**32.** The use of an antibody derivative as claimed in any one of claims 1 to 26 in the preparation of a diagnostic agent.

**33.** An *in vitro* method of diagnosis comprising the use of an antibody derivative as claimed in any one of claims 1 to 26.

**34.** A diagnostic kit comprising an antibody derivative as claimed in any one of claims 1 to 26.

**35.** A method of preparing an antibody derivative as claimed in any one of claims 1 to 26, characterised in that it includes the steps of:
a) treating a first Fc region so as to provide a chemically reactive site thereon, for example, a residue of a linker molecule, and
b) reacting the Fc region with at least one further Fc region directly or via an intervening Fab region.

**36.** A method as claimed in claim 35 characterised in that the first Fc region is part of an Fab Fc construct which has been produced by subjecting to thiol-disulphide interchange an intermediate product containing an open-hinge Fc region having a free sulphydryl group in the hinge region.

**37.** A method as claimed in claim 36, characterised in that it comprises the steps of:
(a) selectively reducing isolated Fab regions of an antibody to form one or more free sulphydryl groups in the hinge region thereof;
(b) derivatising the Fab regions by introduction of a first synthetic chemical linking group reactive with free sulphydryl groups to form a derivatised Fab region which can still be reacted with sulphydryl groups;
(c) reacting the derivatised Fab regions with a molar excess of Fc regions recovered from the same or from a different antibody and having a sulphydryl group in the hinge region, thereby forming a product (FabFc) having one Fc region, one Fab region attached to one Fc chain and a free sulphydryl group at the hinge region of the other Fc chain;
(d) subjecting the FabFc product to thiol-disulphide interchange;
(e) derivatising the thiol-disulphide interchanged FabFc product by introduction of a second synthetic chemical linking group; and
(f) linking the derivatised thiol-disulphide interchanged FabFc product with an FabFc product, prepared by the method of steps (a) to (d) above from the same or from a different antibody and having a sulphydryl group in the hinge region thereby to form bisFabFc in which the antigen binding arms Fab have the same or different specificities.

**38.** A method as claimed in claim 37 characterised in that the Fab and Fc regions are prepared by digestion of starting antibodies with pepsin and papain respectively.

**39.** A method as claimed in claim 37 or claim 38 characterised in that the selective reduction of step (a) of claim 37 is effected by treatment with dithiothreitol.

**40.** A method as claimed in claim 35, characterised in that an Fab region having more than one chemically reactive site is reacted with a molar excess of Fc region to form an antibody derivative with two or more Fc regions linked via the Fab region.

**41.** A method as claimed in claim 40 characterised in that it includes the steps of:
a) disgesting antibody with pepsin;
b) reducing the product to produce a sulphydryl derivative of the antibody fragment so produced;
c) reacting the derivative with a maleimised Fc region; and
d) rendering residual sulphydryl inert.

**42.** A method for the preparation of precursor to an Fc region for use in a method as claimed in any one of claims 35, 40 or 41 characterised in that it includes the steps of:
a) subjecting an Fc region having free sulphydryl groups and an Fab region having a mixed pyridyl disulphide group to disulphide interchange; and
b) rendering any residual sulphydryl groups inert for example by alkylation.

**43.** A precursor to a first Fc region for use in a method as claimed in any one of claims 35, 40, 41 or 42 characterised in that it comprises an Fab region linked via a disulphide bond to an Fc region any residual sulphydryl groups of which have been rendered inert by suitable chemical treatment for example alkylation.

**44.** A method as claimed in claim 42, characterised in that step b) is performed with a suitable alkylating agent, for example N-ethylamaleimide, so as to produce an Fc region with an open hinge configuration.

**45.** A precursor as claimed in claim 43, characterised in that it has an Fc region with an open hinge configuration.

**46.** A method as claimed in claim 42 characterised in that step b) is performed with a suitable alkylating agent for example a bivalent linking group such as o-phenylenedimaleimide so as to produce an Fc region with a closed hinge configuration.

**47.** A precursor as claimed in claim 43 characterised in that it has an Fc region with a closed hinge configuration.

**48.** A method for the preparation of an Fc region for use in a method as claimed in any one of claims 35, 40 or 41 characterised in that it includes the steps of:
a) reducing a product of a method as claimed in any one of claims 42, 44 or 46 or a precursor as claimed in any one of claims 43, 45 or 47 to produce the desired Fc region; and
b) reacting the reduction product so formed with a bivalent linking group for example bis-maleimide.

**49.** A method as claimed in any one of claims 42, 44 or 46 characterised in that it includes the previous steps of:
i) disgesting a first antibody for example a non-human immunoglobulin with pepsin
ii) reduction of the Fab region from step i)
iii) subjecting the reduced Fab region to thiol-disulphide interchange to add a mixed pyridylsulphide group to the Fab region as required for step a) of claim 42; and in parallel with steps i), ii) and iii)
iv) digesting a second antibody for example human immunoglobulin with papain; and
v) reducing the product of step iv) to produce an Fc region having free sulphydryl groups as required for step a) of claim 42.

**50.** A method as claimed in any one of the preceding method claims, characterised in that a reactive site is provided which is a synthetic chemical linking group containing at least two functional groups capable of reacting with free sulphydryl groups, for example maleimide or chloro groups.

**51.** A method as claimed in claim 50 characterised in that the synthetic chemical linking group has three functional groups and is used to link an Fab region with only two cysteine residues in the hinge region.

52. A method as claimed in any one of the preceding method claims, characterised in that the synthetic chemical linking group incorporates a relatively bulky spacer group, for example an inert protein such as plasma albumin.

53. A method as claimed in any one of the preceding method claims, characterised in that the synthetic chemical linking group is residue of a dimaleimide of formula:

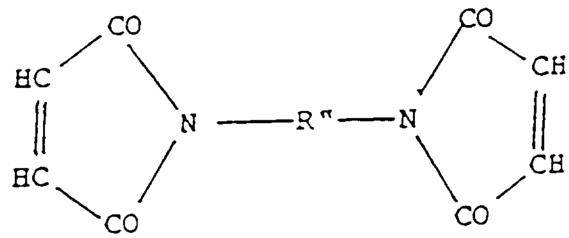

in which R" is a divalent organic radical for example an optionally substituted divalent aryl or aliphatic group such as:
N,N'-o-phenylenedimaleimide or
N,N'-bis(3-maleimidopropionyl)-2-hydroxy-1,3-propanediamine.

**Patentansprüche**

1. Synthetisches Antikörperderivat, welches zumindest zwei Fc-Regionen umfaßt, welche durch kovalente Bindung miteinander verbunden sind (wobei der Begriff "Fc-Region" sowohl Immunglobulin-Fc-Strukturen und aktive Fragmente davon als auch partielle Strukturen umfaßt), dadurch gekennzeichnet, daß die Anzahl an vorhandenen Fab-Regionen die Anzahl an Fc-Regionen nicht überschreitet (wobei der Begriff "Fab-Region" jede Substanz umfaßt, welche Spezifität zu einer Zielzelle aufweist, und jeden Anteil, welcher einer Fab-Region ähnliche funktionelle Eigenschaften aufweist).

2. Antikörperderivat nach Anspruch 1, dadurch gekennzeichnet, daß die kovalente Bindung zumindest eine synthetische chemische Koppelungsgruppe umfaßt.

3. Antikörperderivat nach Anspruch 1 oder Anspruch 2, dadurch gekennzeichnet, daß zumindest ein Teil der kovalenten Bindung zwischen Fc-Regionen im wesentlichen direkt ist.

4. Antikörperderivat nach einem der vorangehenden Ansprüche, dadurch gekennzeichnet, daß es zumindest eine Fab-Region umfaßt.

5. Antikörperderivat nach einem der vorangehenden Ansprüche, dadurch gekennzeichnet, daß es dargestellt wird durch die Formel:

$$Fab(A)\text{-}R\text{-}[\text{-}Fc\text{-}R\text{-}]_m\text{-}Fab(B),$$

wobei
Fab(A) eine Fab-Region aus einem ersten Antikörper A ist;
R eine synthetische chemische Koppelungsgruppe darstellt;
Fc eine Fc-Region darstellt;
m eine Ganzzahl größer als 1 ist; und
Fab (B) eine wahlweise vorhandene Fab-Region aus einem Antikörper B ist, welcher derselbe wie A oder davon unterschiedlich sein kann und welcher in Verwendungsbedingungen funktionell inert sein kann.

6. Antikörperderivat nach Anspruch 5, dadurch gekennzeichnet, daß:
Fab(A) eine Fab-Region aus einem monoklonalen Mausantikörper ist, welche spezifisch auf die schweren Ketten von Human-IgM ist, welcher vorstehend als Anti-Mu bezeichnet wurde;
R aus einer Bismaleimid-Koppelungsgruppe gewonnen wird;

Fc eine Fc-Region ist, welche aus Humanimmunglobulin IgG gewonnen wird;
m gleich 2 ist; und
Fab (B) eine Fab-Region aus einem monoklonalen Mausantikörper ist, welcher auf das CD19-Differenzierungsantigen auf menschlichen B-Lymphozyten spezifisch ist, welcher vorstehend als Anti-CD19 bezeichnet wurde.

**7.** Antikörperderivat nach einem der Ansprüche 1, 2 oder 4 bis 6, dadurch gekennzeichnet, daß zumindest ein Teil der kovalenten Bindung indirekt ist, indem sie eine Fab-Region enthält.

**8.** Antikörperderivat nach Anspruch 7, dadurch gekennzeichnet, daß es dargestellt wird durch die Formel:

Fc-R-Fab(-R-Fc)n,

wobei
Fc eine Fc-Region darstellt;
R eine synthetische chemische Koppelungsgruppe darstellt;
Fab eine Fab-Region darstellt;
n eine Ganzzahl größer gleich 1 ist; und
wobei die Fc's um zusätzliche (-R-Fc)-Gruppen erweitert werden können.

**9.** Antikörperderivat nach Anspruch 8, dadurch gekennzeichnet, daß
Fc eine Fc-Region ist, welche aus Humanimmunglobulin IgG gewonnen wird;
R aus einer Bismaleimid-Koppelungsgruppe gewonnen wird;
Fab eine Fab-Region aus einem monoklonalen Mausantikörper ist, welcher auf das CD37-Differenzierungsantigen auf menschlichen B-Lymphozyten spezifisch ist; und
n gleich 1 ist.

**10.** Antikörperderivat nach einem der vorangehenden Ansprüche, dadurch gekennzeichnet, daß es zumindest eine Fc-Region mit einer offenen Verbindungskonfiguration aufweist.

**11.** Antikörperderivat nach einem der vorangehenden Ansprüche, dadurch gekennzeichnet, daß es zumindest eine Fc-Region mit einer geschlossenen Verbindungskonfiguration aufweist.

**12.** Antikörperderivat nach Anspruch 11, dadurch gekennzeichnet, daß die Konfiguration durch Bindung, umfassend eine synthetische chemische Koppelungsgruppe, geschlossen wird.

**13.** Antikörperderivat nach Anspruch 11, dadurch gekennzeichnet, daß die Konfiguration über eine Disulfidbindung, welche an jedem Ende bei einem Cysteinrest endet, geschlossen wird.

**14.** Antikörperderivat nach einem der Ansprüche 10 bis 13, dadurch gekennzeichnet, daß freie Sulfhydrylgruppen auf den Fc-Regionen beispielsweise durch Alkylierung inertisiert wurden.

**15.** Antikörperderivat nach einem der vorangehenden Ansprüche, dadurch gekennzeichnet, daß es zumindest eine Verbindung zwischen einer Fab-Region und einer Fc-Region umfaßt, wobei die Verbindung eine synthetische chemische Koppelungsgruppe umfaßt.

**16.** Antikörperderivat nach einem der vorangehenden Ansprüche, dadurch gekennzeichnet, daß zumindest eine der Fc-Regionen aus Humanimmunglobulin gewonnen wird, beispielsweise aus IgG oder IgG1.

**17.** Antikörperderivat nach einem der vorangehenden Ansprüche, dadurch gekennzeichnet, daß es eine Fab-Region aufweist, welche zumindest zum Teil aus nichtmenschlichem Immunglobulin gewonnen wird.

**18.** Antikörperderivat nach einem der vorangehenden Ansprüche, dadurch gekennzeichnet, daß es zumindest eine Fab-Region aufweist, welche aus monoklonalem Antikörper gewonnen wurde.

**19.** Antikörperderivat nach einem der vorangehenden Ansprüche, dadurch gekennzeichnet, daß es zumindest eine synthetische chemische Koppelungsgruppe aufweist, welche an ihrem einen Ende über eine

Thioetherbindung an einen Cysteinrest in einer Fab-Region oder einer Fc-Region und an ihrem anderen Ende an einen Cysteinrest in einer Fc-Region gebunden ist.

**20.** Antikörperderivat nach einem der vorangehenden Ansprüche, dadurch gekennzeichnet, daß es zumindest eine synthetische chemische Koppelungsgruppe aufweist, welche einen Rest eines divalenten oder trivalenten Linker-Moleküls umfaßt.

**21.** Antikörperderivat nach Anspruch 20, dadurch gekennzeichnet, daß der Rest ein divalenter Rest eines Bismaleimids ist.

**22.** Antikörperderivat nach Anspruch 21, dadurch gekennzeichnet, daß das Bismaleimid ein Dimaleimid mit der Formel:

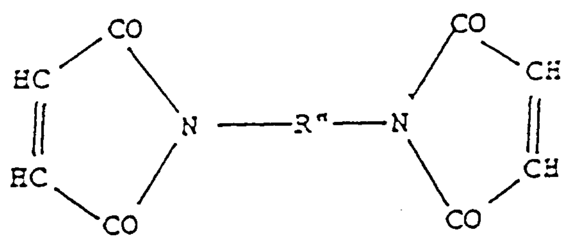

ist, wobei R" ein divalentes organisches Radikal, beispielsweise eine gegebenenfalls substituierte Aryl- oder Alkylgruppe, ist.

**23.** Antikörperderivat nach Anspruch 20, dadurch gekennzeichnet, daß der Rest ein Rest eines trivalenten Linker-Moleküls ist, welcher eine Fab-Region, die zwei Cysteinreste aufweist, mit einer Fc-Region verbindet.

**24.** Antikörperderivat nach Anspruch 23, dadurch gekennzeichnet, daß der Rest ein Rest von Tris-(2-chlorethyl)amin ist.

**25.** Antikörperderivat nach einem der vorangehenden Ansprüche, dadurch gekennzeichnet, daß es zumindest eine synthetische chemische Koppelungsgruppe aufweist, welche ein relativ voluminöses Abstandhaltermolekül umfaßt, um den Abstand, in dem jene Teile des Antikörpers getrennt sind, welche durch sie verbunden werden, zu vergrößern.

**26.** Antikörperderivat nach Anspruch 25, dadurch gekennzeichnet, daß das Abstandhaltermolekül aus einem trägen Protein gewonnen wird, beispielsweise aus Plasmaalbumin.

**27.** Pharmazeutisches Präparat, dadurch gekennzeichnet, daß es als Wirkprinzip ein Antikörperderivat umfaßt, wie es in einem der vorangehenden Ansprüche vorgesehen wird, mit einem pharmazeutisch akzeptablen Verdünnungsmittel oder Träger.

**28.** Verwendung eines Antikörperderivats nach einem der Ansprüche 1 bis 26 zum Herstellen eines Mittels zum Behandeln oder Verhindern von krebsartigen Zuständen.

**29.** Verwendung eines Antikörperderivats nach einem der Ansprüche 1 bis 26 zum Herstellen eines Mittels zum Abtöten von Säugetierzellen, beispielsweise von Zellen, welche mit einem Virus oder einem anderen intrazellulären Mikroben infiziert sind, oder von mißgebildeten Autoimmunzellen, beispielsweise abnormen Lymphozytklonen, welche eine Autoimmunkrankheit hervorrufen.

**30.** Verwendung eines Antikörperderivats nach einem der Ansprüche 1 bis 26 zum Herstellen eines Mittels zum Entfernen unerwünschter Zellen aus Knochenmark.

**31.** Verwendung eines Antikörperderivats nach einem der Ansprüche 1 bis 26 zum Herstellen eines Mittels für die Immunsuppression in Transplantatempfängern.

**32.** Verwendung eines Antikörperderivats nach einem der Ansprüche 1 bis 26 bei der Herstellung eines Diagnostikmittels.

**33.** In vitro-Diagnoseverfahren, umfassend die Verwendung eines Antikörperderivats nach einem der Ansprüche 1 bis 26.

**34.** Diagnoseset, welches ein Antikörperderivat nach einem der Ansprüche 1 bis 26 enthält.

**35.** Verfahren zur Herstellung eines Antikörperderivats nach einem der Ansprüche 1 bis 26, dadurch gekennzeichnet, daß es die folgenden Schritte umfaßt:

a) Behandeln einer ersten Fc-Region, um eine chemisch reaktive Stelle darauf zu schaffen, beispielsweise einen Rest eines Linker-Moleküls; und

b) Reagieren der Fc-Region mit zumindest einer weiteren Fc-Region direkt oder über eine dazwischentretende Fab-Region.

**36.** Verfahren nach Anspruch 35, dadurch gekennzeichnet, daß die erste Fc-Region ein Teil eines Fab-Fc-Konstrukts ist, welches erzeugt wurde, indem ein Zwischenprodukt, welches eine offene Fc-Verbindungsregion mit einer freien Sulfhydrylgruppe in der Verbindungsregion enthielt, einem Thiol-Disulfid-Austausch unterzogen wurde.

**37.** Verfahren nach Anspruch 36, dadurch gekennzeichnet, daß es die folgenden Schritte umfaßt:

(a) Selektives Reduzieren isolierter Fab-Regionen eines Antikörpers, um in der Verbindungsregion davon eine oder mehrere freie Sulfhydrylgruppen zu bilden.

(b) Derivatisieren der Fab-Regionen durch Einbringen einer ersten synthetischen chemischen Koppelungsgruppe, welche mit freien Sulfhydrylgruppen reaktiv ist, um eine derivatisierte Fab-Region zu bilden, die noch mit Sulfhydrylgruppen zur Reaktion gebracht werden kann.

(c) Reagieren der derivatisierten Fab-Regionen mit einem Molarüberschuß an Fc-Regionen, welche aus demselben oder einem anderen Antikörper gewonnen wurden und eine Sulfhydrylgruppe in der Verbindungsregion aufweisen, wodurch ein Produkt (FabFc) herstellt wird, welches eine Fc-Region, eine Fab-Region, welche an einer Fc-Kette befestigt ist, und eine freie Sulfhydrylgruppe an der Verbindungsregion der anderen Fc-Kette aufweist;

(d) Unterziehen des FabFc-Produkts einem Thiol-Disulfidaustausch;

(e) Derivatisieren des einem Thiol-Disulfid-Austausch unterzogenen FabFc-Produkts durch Einbringen einer zweiten synthetischen chemischen Koppelungsgruppe; und

(f) Verbinden des derivatisierten, einem Thiol-Disulfid-Austausch unterzogenen FabFc-Produkts mit einem FabFc-Produkt, welches durch das Verfahren der oben genannten Schritte (a) bis (d) aus demselben oder einem anderen Antikörper hergestellt wurde und eine Sulfhydrylgruppe in der Verbindungsregion aufweist, um dadurch BisFabFc zu bilden, worin die Antigenbindearme Fab dieselben oder andere Spezifitäten besitzen.

**38.** Verfahren nach Anspruch 37, dadurch gekennzeichnet, daß die Fab- und Fc-Regionen durch Aufschließen der Ausgangsantikörper mit Pepsin bzw. Papain hergestellt werden.

**39.** Verfahren nach Anspruch 37 oder Anspruch 38, dadurch gekennzeichnet, daß die selektive Reduktion aus Schritt (a) von Anspruch 37 durch Behandlung mit Dithiothreitol realisiert wird.

**40.** Verfahren nach Anspruch 35, dadurch gekennzeichnet, daß eine Fab-Region, welche mehr als eine chemisch reaktive Stelle aufweist, mit einem Molarüberschuß an Fc-Region reagiert wird, um ein Antikörperderivat mit zwei oder mehreren Fc-Regionen zu bilden, welche über die Fab-Region verbunden sind.

**41.** Verfahren nach Anspruch 40, dadurch gekennzeichnet, daß es die folgenden Schritte umfaßt:

a) Aufschließen des Antikörpers mit Pepsin;

b) Reduzieren des Produktes, um ein Sulfhydrylderivat des auf diese Weise hergestellten Antikörperfragments herzustellen;

c) Reagieren des Derivats mit einer maleimisierten Fc-Region; und

d) Intertisieren des Restsulfhydryls.

**42.** Verfahren zum Herstellen eines Vorläufers für eine Fc-Region zur Verwendung in einem Verfahren nach einem der Ansprüche 35, 40, 41, dadurch gekennzeichnet, daß es die folgenden Schritte umfaßt:
a) Unterziehen einer Fc-Region mit freien Sulfhydrylgruppen und eine Fab-Region mit einer gemischten Pyridyldisulfidgruppe einem Disulfidaustausch; und
b) Intertisieren aller Restsulihydrylgruppen, beispielsweise durch Alkylierung.

**43.** Vorläufer für eine erste Fc-Region zur Verwendung in einem Verfahren nach einem der Ansprüche 35, 40, 41 oder 42, dadurch gekennzeichnet, daß er eine Fab-Region umfaßt, welche über eine Disulfidbindung an eine Fc-Region gebunden ist, wobei alle Restsulfhydrylgruppen davon durch geeignete chemische Behandlung, beispielsweise Alkylierung, intertisiert wurden.

**44.** Verfahren nach Anspruch 42, dadurch gekennzeichnet, daß Schritt b) mit einem geeigneten Alkyliermittel, beispielsweise N-Ethylamaleimid, durchgeführt wird, um eine Fc-Region mit einer offenen Verbindungskonfiguration zu erzeugen.

**45.** Vorläufer nach Anspruch 43, dadurch gekennzeichnet, daß er eine Fc-Region mit einer offenen Verbindungskonfiguration aufweist.

**46.** Verfahren nach Anspruch 42, dadurch gekennzeichnet, daß Schritt b) mit einem geeigneten Alkyliermittel, beispielsweise einer bivalenten Koppelungsgruppe wie o-Phenylendimaleimid, durchgeführt wird, um eine Fc-Region mit einer geschlossenen Verbindungskonfiguration zu erzeugen.

**47.** Vorläufer nach Anspruch 43, dadurch gekennzeichnet, daß er eine Fc-Region mit einer geschlossenen Verbindungskonfiguration aufweist.

**48.** Verfahren zur Herstellung einer Fc-Region zur Verwendung in einem Verfahren nach einem der Ansprüche 35, 40 oder 41, dadurch gekennzeichnet, daß es die folgenden Schritte umfaßt:
a) Reduzieren eines Produkts aus einem Verfahren nach einem der Ansprüche 42, 44 oder 46 oder eines Vorläufers nach einem der Ansprüche 43, 45 oder 47, um die gewünschte Fc-Region zu erzeugen; und
b) Reagieren des derart hergestellten Reduktionsprodukts mit einer bivalenten Koppelungsgruppe, beispielsweise mit Bismaleimid.

**49.** Verfahren nach einem der Ansprüche 42, 44 oder 46, dadurch gekennzeichnet, daß es die vorangehenden Schritte umfaßt:
i) Aufschließen eines ersten Antikörpers, beispielsweise eines nichtmenschlichen Immunglobulins mit Pepsin;
ii) Reduktion der Fab-Region aus Schritt i);
iii) Unterziehen der reduzierten Fab-Region einem Thiol-Disulfid-Austausch, um der Fab-Region eine gemischte Pyridylsulfidgruppe beizugeben, wie für Schritt a) aus Anspruch 42 erforderlich ist;
und parallel zu den Schritten i), ii) und iii)
iv) Aufschließen eines zweiten Antikörpers, beispielsweise von Humanglobulin, mit Papain; und
v) Reduzieren des Produkts aus Schritt iv), um eine Fc-Region herzustellen, welche freie Sulfhydrylgruppen aufweist, wie für Schritt a) aus Anspruch 42 erforderlich ist.

**50.** Verfahren nach einem der vorangehenden Verfahrensansprüche, dadurch gekennzeichnet, daß eine reaktive Stelle vorgesehen wird, welche eine synthetische chemische Koppelungsgruppe ist, welche zumindest zwei funktionelle Gruppen enthält, die in der Lage sind, mit freien Sulfhydrylgruppen zu reagieren, beispielsweise Maleimid oder Chlor-Gruppen.

**51.** Verfahren nach Anspruch 50, dadurch gekennzeichnet, daß die synthetische chemische Koppelungsgruppe drei funktionelle Gruppen aufweist und verwendet wird, um eine Fab-Region mit nur zwei Cysteinresten in der Verbindungsregion zu verbinden.

**52.** Verfahren nach einem der vorangehenden Verfahrensansprüche, dadurch gekennzeichnet, daß die synthetische chemische Koppelungsgruppe eine relativ voluminöse Abstandhaltergruppe einbindet, beispielsweise ein inertes Protein wie Plasmaalbumin.

**53.** Verfahren nach einem der vorangehenden Verfahrensansprüche, dadurch gekennzeichnet, daß die synthetische chemische Koppelungsgruppe ein Rest eines Dimaleimids mit der Formel:

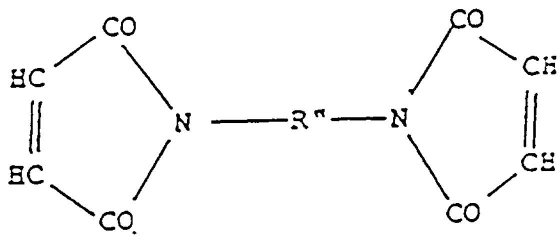

ist, bei welchem R" ein divalentes organisches Radikal, beispielsweise ein gegebenenfalls substituiertes divalentes Aryl oder eine aliphatische Gruppe, ist, beispielsweise:
N,N'-o-Phenylendimaleimid oder
N,N'-Bis(3-maleimidoproionyl)-2-hydroxy-1,3-propandiamin.

## Revendications

**1.** Dérivé synthétique d'anticorps, qui comprend au moins deux régions Fc jointes par une liaison covalente (où le terme "région Fc" comprend les structures Fc d'une immunoglobuline, les fragments actifs de celle-ci et les structures partielles), caractérisé en ce que le nombre de régions Fab présentes n'excède pas le nombre de régions Fc (où le terme "région Fab" comprend toute entité présentant une spécificité pour une cellule cible et toute fraction présentant des propriétés fonctionnelles similaires à une région Fab).

**2.** Dérivé d'anticorps suivant la revendication 1, caractérisé en ce que la liaison covalente comprend au moins un radical de liaison chimique synthétique.

**3.** Dérivé d'anticorps suivant la revendication 1 ou 2, caractérisé en ce qu'au moins une partie de la liaison covalente entre les régions Fc est essentiellement directe.

**4.** Dérivé d'anticorps suivant l'une quelconque des revendications précédentes, caractérisé en ce qu'il comprend au moins une région Fab.

**5.** Dérivé d'anticorps suivant l'une quelconque des revendications précédentes, caractérisé en ce qu'il est représenté par la formule :

$$Fab(A)\text{-}R\text{-}[\text{-}Fc\text{-}R\text{-}]_m\text{-}Fab(B)$$

où :
Fab(A) est une région Fab provenant d'un premier anticorps A;
R représente un radical de liaison chimique synthétique;
Fc représente une région Fc;
m est un entier supérieur à 1, et
Fab(B) est une région Fab, facultativement présente, provenant d'un anticorps B qui peut être le même ou différent de l'anticorps A et qui peut être fonctionnellement inerte dans les conditions d'utilisation.

**6.** Dérivé d'anticorps suivant la revendication 5, caractérisé en ce que :
Fab(A) est une région Fab d'un anticorps monoclonal de souris spécifique des chaînes lourdes de l'IgM humaine, noté ici anti-mu;
R provient d'un radical de liaison bismaléimide;
Fc est une région Fc provenant d'une immunoglobuline IgG humaine;
m égale 2, et
Fab(B) est une région Fab provenant d'un anticorps monoclonal de souris spécifique de l'antigène de différentiation CD19 sur les lymphocytes B humains, noté ici anti-CD19.

7. Dérivé d'anticorps suivant l'une quelconque des revendications 1, 2 ou 4 à 6, caractérisé en ce qu'au moins une partie de la liaison covalente est indirecte en ce qu'elle comprend une région Fab.

8. Dérivé d'anticorps suivant la revendication 7, caractérisé en ce qu'il est représenté par la formule :

Fc-R-Fab(-R-Fc)$_n$

où :
Fc représente une région Fc;
R représente un radical de liaison chimique synthétique;
Fab représente une région Fab;
n est un entier égal ou supérieur à 1, et où :
les Fc peuvent être, en outre, prolongés par des radicaux (-R-Fc) supplémentaires.

9. Dérivé d'anticorps suivant la revendication 8, caractérisé en ce que :
Fc est une région Fc provenant d'une immunoglobuline IgG humaine;
R provient d'un radical de liaison bismaléimide;
Fab est une région Fab provenant d'un anticorps monoclonal de souris spécifique de l'antigène de différentiation CD37 sur les lymphocytes B humains, et
n égale 1.

10. Dérivé d'anticorps suivant l'une quelconque des revendications précédentes, caractérisé en ce qu'il présente au moins une région Fc avec une configuration de charnière ouverte.

11. Dérivé d'anticorps suivant l'une quelconque des revendications précédentes, caractérisé en ce qu'il présente au moins une région Fc avec une configuration de charnière fermée.

12. Dérivé d'anticorps suivant la revendication 11, caractérisé en ce que la configuration est fermée par un lien comprenant un radical de liaison chimique synthétique.

13. Dérivé d'anticorps suivant la revendication 11, caractérisé en ce que la configuration est fermée par un lien disulfure aboutissant à chaque extrémité à un résidu cystéine.

14. Dérivé d'anticorps suivant l'une quelconque des revendications 10 à 13, caractérisé en ce que les radicaux sulfhydryle libres sur les régions Fc ont été rendus inertes, par exemple par alcoylation.

15. Dérivé d'anticorps suivant l'une quelconque des revendications précédentes, caractérisé en ce qu'il comprend au moins une liaison entre une région Fab et une région Fc, la liaison comprenant un radical de liaison chimique synthétique.

16. Dérivé d'anticorps suivant l'une quelconque des revendications précédentes, caractérisé en ce qu'au moins une des régions Fc provient d'une immunoglobuline humaine, par exemple IgG ou IgG1.

17. Dérivé d'anticorps suivant l'une quelconque des revendications précédentes, caractérisé en ce qu'il présente une région Fab dont au moins une partie de celle-ci provient d'une immunoglobuline non humaine.

18. Dérivé d'anticorps suivant l'une quelconque des revendications précédentes, caractérisé en ce qu'il présente au moins une région Fab provenant d'un anticorps monoclonal.

19. Dérivé d'anticorps suivant l'une quelconque des revendications précédentes, caractérisé en ce qu'il présente au moins un radical de liaison chimique synthétique, lié par une liaison thioéther à un résidu cystéine d'une région Fab ou d'une région Fc à une extrémité, et à un résidu cystéine d'une région Fc à l'autre extrémité du radical de liaison.

20. Dérivé d'anticorps suivant l'une quelconque des revendications précédentes, caractérisé en ce qu'il présente au moins un radical de liaison chimique synthétique, qui comprend un résidu d'une molécule de liaison divalente ou trivalente.

**21.** Dérivé d'anticorps suivant la revendication 20, caractérisé en ce que le résidu est un résidu divalent d'un bismaléimide.

**22.** Dérivé d'anticorps suivant la revendication 21, caractérisé en ce que le bismaléimide est un dimaléimide de formule :

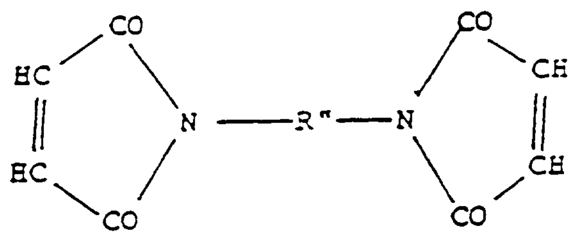

où :

R" est un radical organique divalent, par exemple, un radical aryle ou alcoyle facultativement substitué.

**23.** Dérivé d'anticorps suivant la revendication 20, caractérisé en ce que le résidu est un résidu d'une molécule de liaison trivalente, liant une région Fab ayant deux résidus cystéine à une région Fc.

**24.** Dérivé d'anticorps suivant la revendication 23, caractérisé en ce que le résidu est un résidu d'une tris-(2-chloroéthyl)amine.

**25.** Dérivé d'anticorps suivant l'une quelconque des revendications précédentes, caractérisé en ce qu'il présente au moins un radical de liaison chimique synthétique, qui comprend une molécule d'espacement relativement encombrante pour augmenter la distance séparant les parties de l'anticorps qui sont liées par celui-ci.

**26.** Dérivé d'anticorps suivant la revendication 25, caractérisé en ce que la molécule d'espacement provient d'une protéine inerte, par exemple de l'albumine plasmatique.

**27.** Préparation pharmaceutique caractérisée en ce qu'elle comprend comme principe actif, un dérivé d'anticorps suivant l'une quelconque des revendications précédentes, avec un diluant ou excipient pharmaceutiquement acceptable.

**28.** Utilisation d'un dérivé d'anticorps suivant l'une quelconque des revendications 1 à 26, pour la préparation d'un agent pour le traitement ou la prévention d'états cancéreux.

**29.** Utilisation d'un dérivé d'anticorps suivant l'une quelconque des revendications 1 à 26, pour la préparation d'un agent pour tuer des cellules de mammifères, par exemple, des cellules infectées par un virus ou un autre microbe intracellulaire, ou des cellules malignes autoimmunes telles que des clones lymphocytaires anormaux provoquant une maladie autoimmune.

**30.** Utilisation d'un dérivé d'anticorps suivant l'une quelconque des revendications 1 à 26, pour la préparation d'un agent pour purger des cellules non souhaitées de la moelle osseuse.

**31.** Utilisation d'un dérivé d'anticorps suivant l'une quelconque des revendications 1 à 26, pour la préparation d'un agent pour l'immunosuppression de récepteurs de greffe.

**32.** Utilisation d'un dérivé d'anticorps suivant l'une quelconque des revendications 1 à 26, dans la préparation d'un agent de diagnostic.

**33.** Procédé in vitro de diagnostic comprenant l'utilisation d'un dérivé d'anticorps suivant l'une quelconque des revendications 1 à 26.

**34.** Equipement diagnostique comprenant un dérivé d'anticorps suivant l'une quelconque des revendications 1 à 26.

**35.** Procédé de préparation d'un dérivé d'anticorps suivant l'une quelconque des revendications 1 à 26, caractérisé en ce qu'il comprend les étapes de :
a) traitement d'une première région Fc afin de fournir un site chimiquement réactif sur celui-ci, par exemple, un résidu d'une molécule de liaison, et
b) réaction de la région Fc avec au moins une autre région Fc, directement ou par une région Fab intermédiaire.

**36.** Procédé suivant la revendication 35, caractérisé en ce que la première région Fc fait partie d'une construction FabFc, qui a été produite en soumettant à un échange thiol-disulfure, un produit intermédiaire contenant une région Fc à charnière ouverte ayant un radical sulfhydryle libre sur la région charnière.

**37.** Procédé suivant la revendication 36, caractérisé en ce qu'il comprend les étapes de :
a) réduction sélective des régions Fab isolées d'un anticorps, pour former un ou plusieurs radicaux sulfhydryle libres dans la région charnière de celles-ci;
b) dérivatisation des régions Fab par introduction d'un premier radical de liaison chimique synthétique, réactif avec les radicaux sulfhydryle libres pour former une région Fab dérivatisée qui peut ensuite réagir avec des radicaux sulfhydryle;
c) réaction des régions Fab dérivatisées avec un excès molaire de région Fc provenant du même anticorps ou d'un anticorps différent et ayant un radical sulfhydryle dans la région charnière, pour former ainsi un produit (FabFc) ayant une région Fc, une région Fab attachée à une chaîne Fc et un radical sulfhydryle libre à la région charnière de l'autre chaîne Fc;
d) réalisation d'un échange thiol-disulfure sur le produit FabFc;
e) dérivatisation du produit FabFc ayant subi l'échange thiol-disulfure, par introduction d'un deuxième radical de liaison chimique synthétique, et
f) liaison du produit FabFc ayant subi l'échange thiol-disulfure, dérivatisé, avec un produit FabFc, préparé par le procédé des étapes a) à d) ci-dessus, à partir du même anticorps ou d'un anticorps différent et ayant un radical sulfhydryle dans la région charnière de celui-ci, pour former un bisFabFc dans lequel les bras de liaison d'antigène Fab ont des spécificités identiques ou différentes.

**38.** Procédé suivant la revendication 37, caractérisé en ce que les régions Fab et Fc sont préparées par digestion d'anticorps de départ par de la pepsine et de la papaïne, respectivement.

**39.** Procédé suivant la revendication 37 ou 38, caractérisé en ce que la réduction sélective de l'étape a) de la revendication 37 est réalisée par traitement avec du dithiothréitol.

**40.** Procédé suivant la revendication 35, caractérisé en ce qu'une région Fab ayant plus d'un site chimiquement réactif est mise à réagir avec un excès molaire de région Fc, pour former un dérivé d'anticorps avec deux ou plusieurs régions Fc liées via la région Fab.

**41.** Procédé suivant la revendication 40, caractérisé en ce qu'il comprend les étapes de :
a) digestion de l'anticorps par de la pepsine;
b) réduction du produit pour produire un dérivé sulfhydryle du fragment d'anticorps ainsi produit;
c) réaction du dérivé avec une région Fc maléimisée, et
d) transformation du résidu sulfhydryle en un résidu inerte.

**42.** Procédé de préparation d'un précurseur d'une région Fc, à utiliser dans un procédé suivant l'une quelconque des revendications 35, 40 ou 41, caractérisé en ce qu'il comprend les étapes de :
a) réalisation d'un échange de disulfure entre une région Fc ayant des radicaux sulfhydryle libres et une région Fab ayant un radical pyridyle-disulfure mixte, et
b) transformation de tous les résidus sulfhydryle résiduels en résidus inertes, par exemple, par alcoylation.

**43.** Précurseur d'une première région Fc à utiliser dans un procédé suivant l'une quelconque des revendications 35, 40, 41 ou 42, caractérisé en ce qu'il comprend une région Fab liée par un lien disulfure à une région Fc, tout radical sulfhydryle résiduel ayant été rendu inerte par un traitement chimique approprié, par exemple, une alcoylation.

**44.** Procédé suivant la revendication 42, caractérisé en ce que l'étape b) est réalisée avec un agent d'alcoylation approprié, par exemple, le N-éthylmaléimide, afin de produire une région Fc avec une configuration de charnière ouverte.

**45.** Précurseur suivant la revendication 43, caractérisé en ce qu'il présente une région Fc avec une configuration de charnière ouverte.

**46.** Procédé suivant la revendication 42, caractérisé en ce que l'étape b) est réalisée avec un agent d'alcoylation approprié, par exemple un radical de liaison bivalent tel que le o-phénylènedimaléimide, afin de produire une région Fc avec une configuration de charnière fermée.

**47.** Précurseur suivant la revendication 43, caractérisé en ce qu'il présente une région Fc avec une configuration de charnière fermée.

**48.** Procédé de préparation d'une région Fc à utiliser dans un procédé suivant l'une quelconque des revendications 35, 40 ou 41, caractérisé en ce qu'il comprend les étapes de :

a) réduction d'un produit d'un procédé suivant l'une quelconque des revendications 42, 44 ou 46 ou d'un précurseur suivant l'une quelconque des revendications 43, 45 ou 47 pour produire la région Fc souhaitée, et

b) réaction du produit de réduction ainsi formé avec un radical de liaison bivalent, par exemple, un bismaléimide.

**49.** Procédé suivant l'une quelconque des revendications 42, 44 ou 46, caractérisé en ce qu'il comprend les étapes précédentes de :

i) digestion d'un premier anticorps, par exemple, une immunoglobuline non humaine, par la pepsine;

(ii) réduction de la région Fab de l'étape i);

(iii) soumission de la région Fab réduite à un échange thiol-disulfure pour ajouter un radical pyridylesulfure mixte à la région Fab, comme nécessité par l'étape a) de la revendication 42,

et en parallèle aux étapes i), ii) et iii);

iv) digestion d'un deuxième anticorps, par exemple une immunoglobuline humaine, par de la papaïne, et

v) réduction du produit de l'étape iv) pour produire une région Fc ayant des radicaux sulfhydryle libres comme nécessité par l'étape a) de la revendication 42.

**50.** Procédé suivant l'une quelconque des revendications de procédé précédentes, caractérisé en ce qu'un site réactif est fourni, qui est un radical de liaison chimique synthétique contenant au moins deux radicaux fonctionnels capables de réagir avec les radicaux sulfhydryle libres, par exemple, des radicaux maléimide ou chloro.

**51.** Procédé suivant la revendication 50, caractérisé en ce que le radical de liaison chimique synthétique présente trois radicaux fonctionnels et est utilisé pour lier une région Fab avec seulement deux résidus cystéine dans la région charnière.

**52.** Procédé suivant l'une quelconque des revendications de procédé précédentes, caractérisé en ce que le radical de liaison chimique synthétique incorpore un radical d'espacement relativement encombrant, par exemple une protéine inerte telle que l'albumine plasmatique.

**53.** Procédé suivant l'une quelconque des revendications précédentes, caractérisé en ce que le radical de liaison chimique synthétique est le résidu d'un dimaléimide de formule :

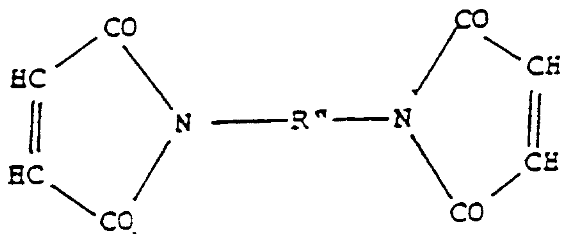

où :

R" est un radical organique divalent, par exemple, un radical aryle ou aliphatique divalent facultativement substitué tel que :

un N,N'-o-phénylènedimaléimide, ou

une N,N'-bis(3-maléimidopropionyl)-2-hydroxy-1,3-propanediamine.

*Fig. 1.*

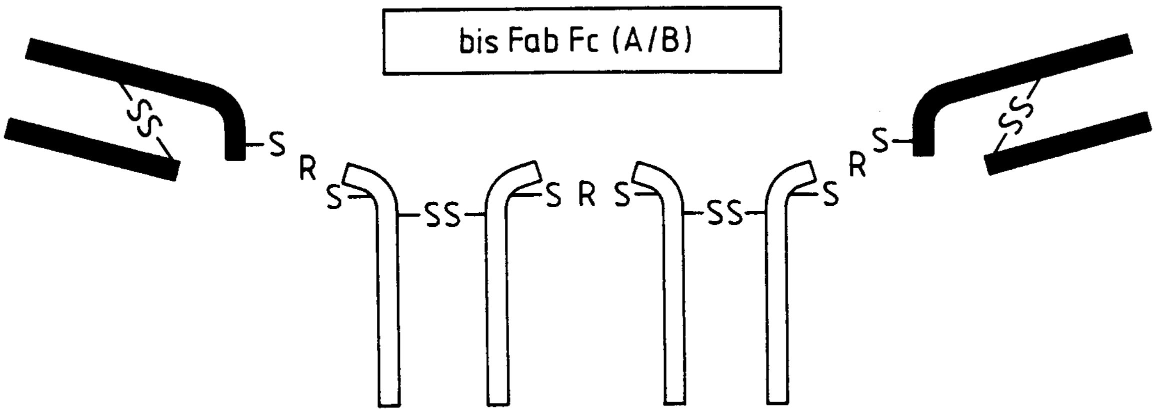
bis Fab Fc (A/B)
SS
S
R
S
SS
S R S
SS
S
R
S
SS

*Fig. 3.*

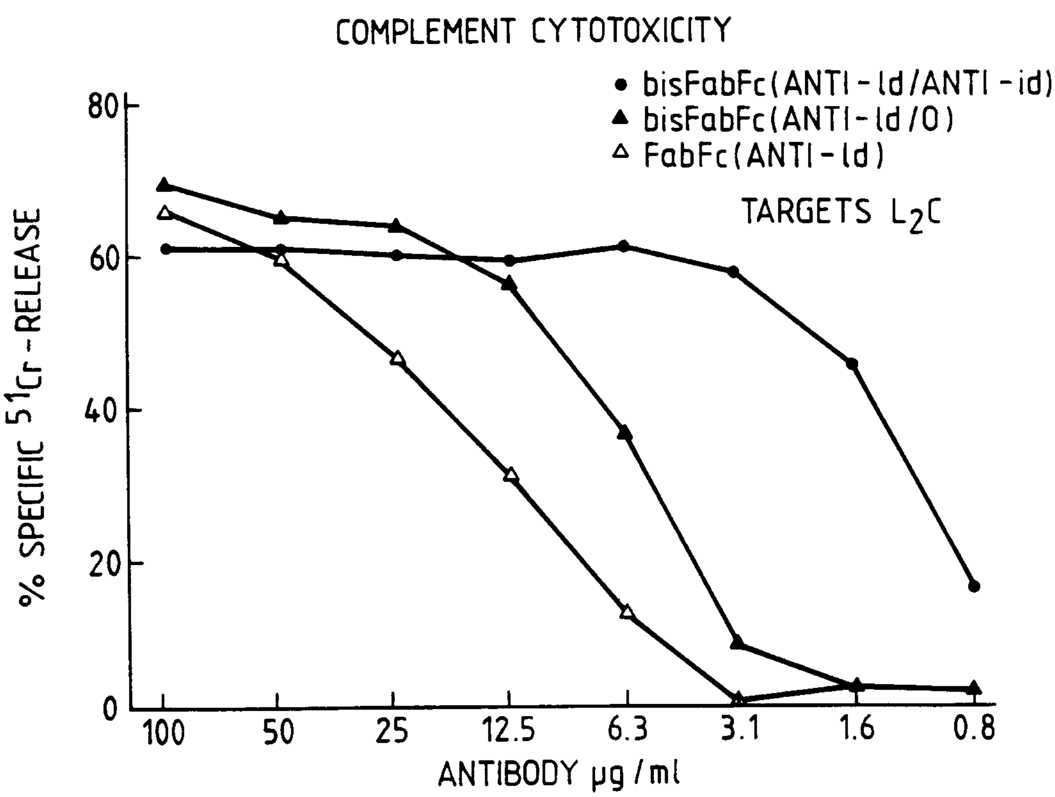
COMPLEMENT CYTOTOXICITY
● bisFabFc(ANTI-Id/ANTI-id)
▲ bisFabFc(ANTI-Id/O)
△ FabFc(ANTI-Id)
TARGETS L2C
% SPECIFIC 51Cr-RELEASE
80
60
40
20
0
100
50
25
12.5
6.3
3.1
1.6
0.8
ANTIBODY μg/ml

Fig.2.

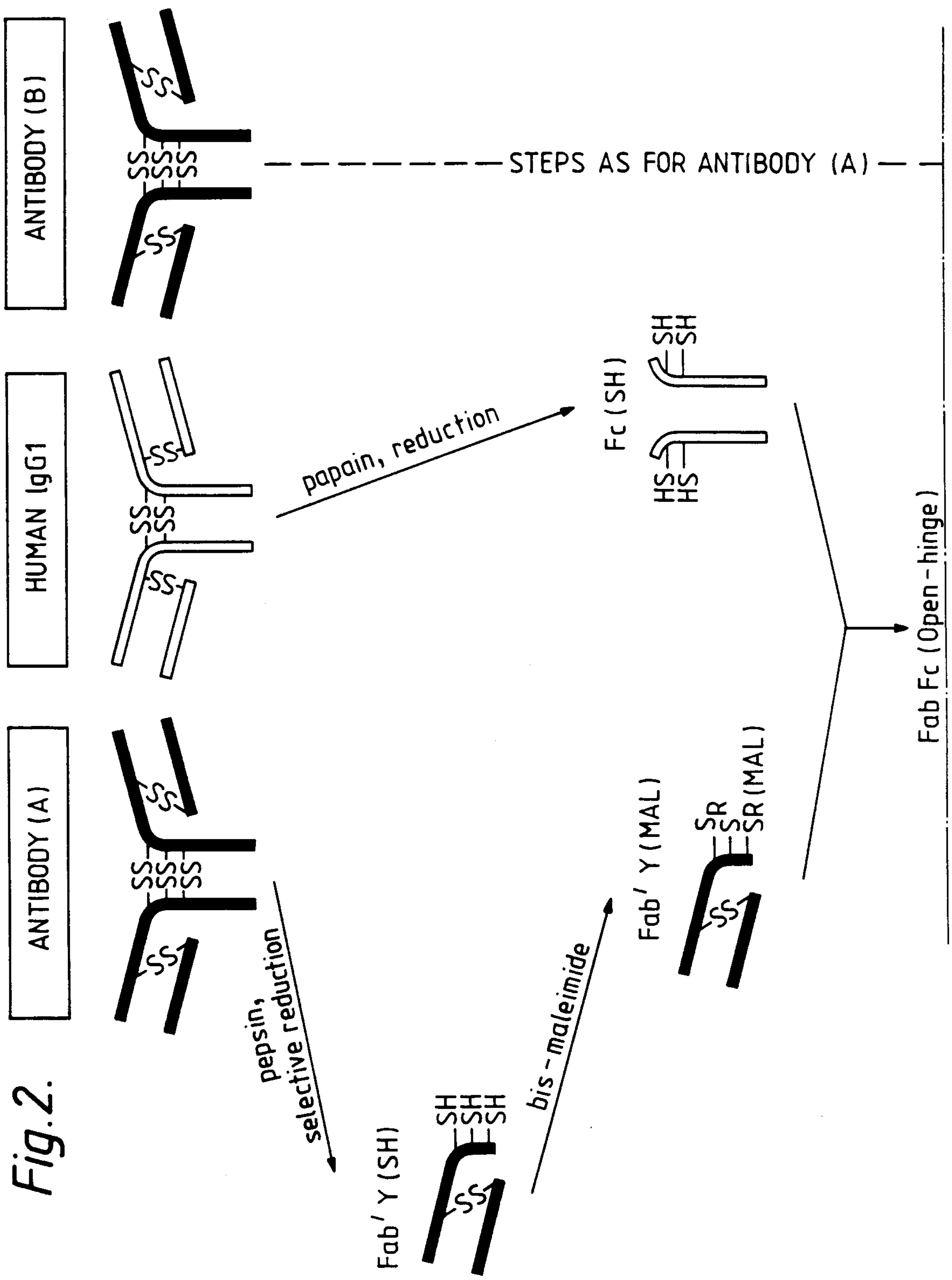
ANTIBODY (A)
HUMAN IgG1
ANTIBODY (B)
STEPS AS FOR ANTIBODY (A)
pepsin, selective reduction
papain, reduction
Fab' Y (SH)
bis-maleimide
Fab' Y (MAL)
Fc (SH)
Fab Fc (Open-hinge)

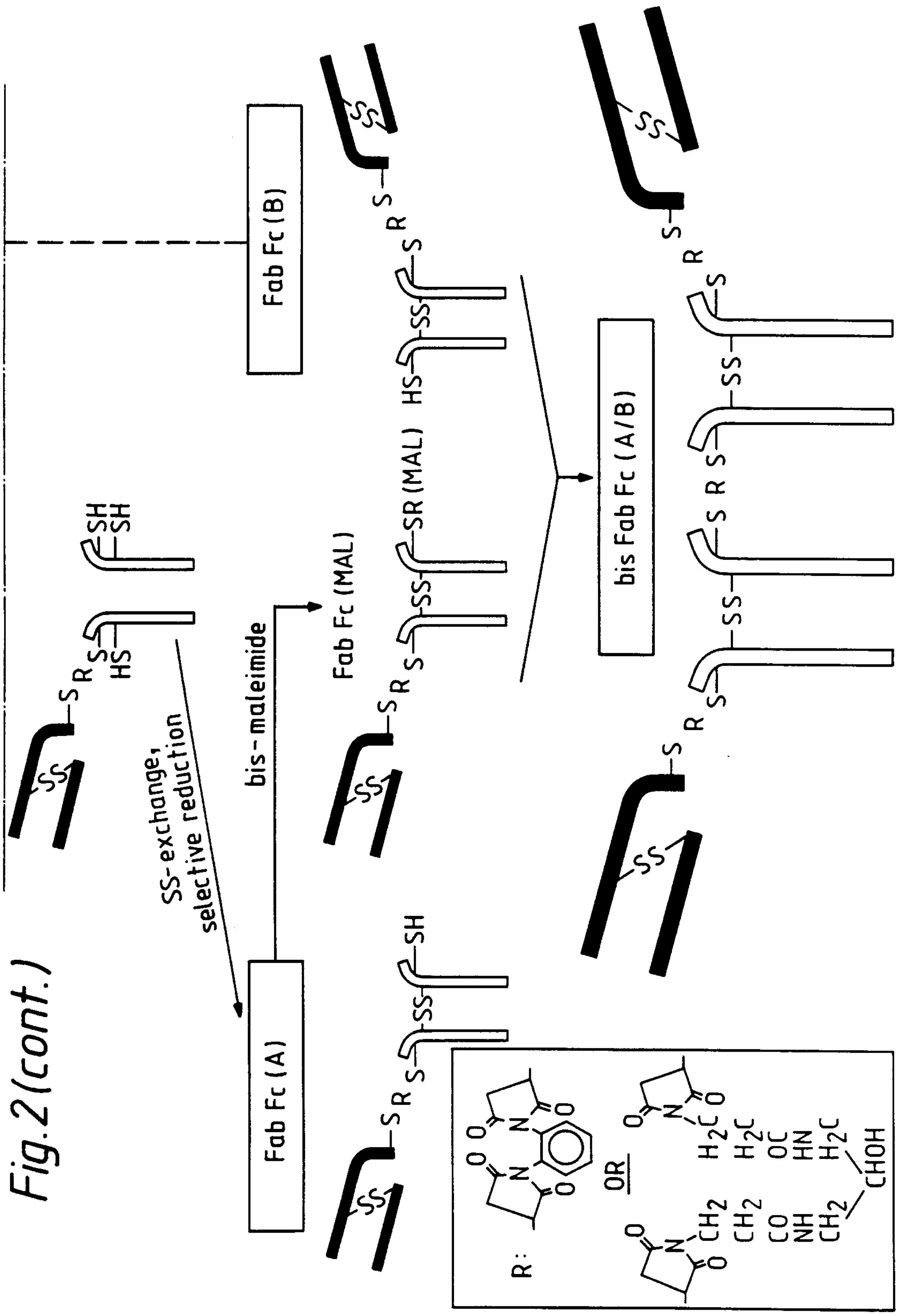
Fab Fc (A)
Fab Fc (B)
SS-exchange, selective reduction
bis-maleimide
Fab Fc (MAL)
bis Fab Fc (A/B)

Fig.2(cont.)

Fig.4A.

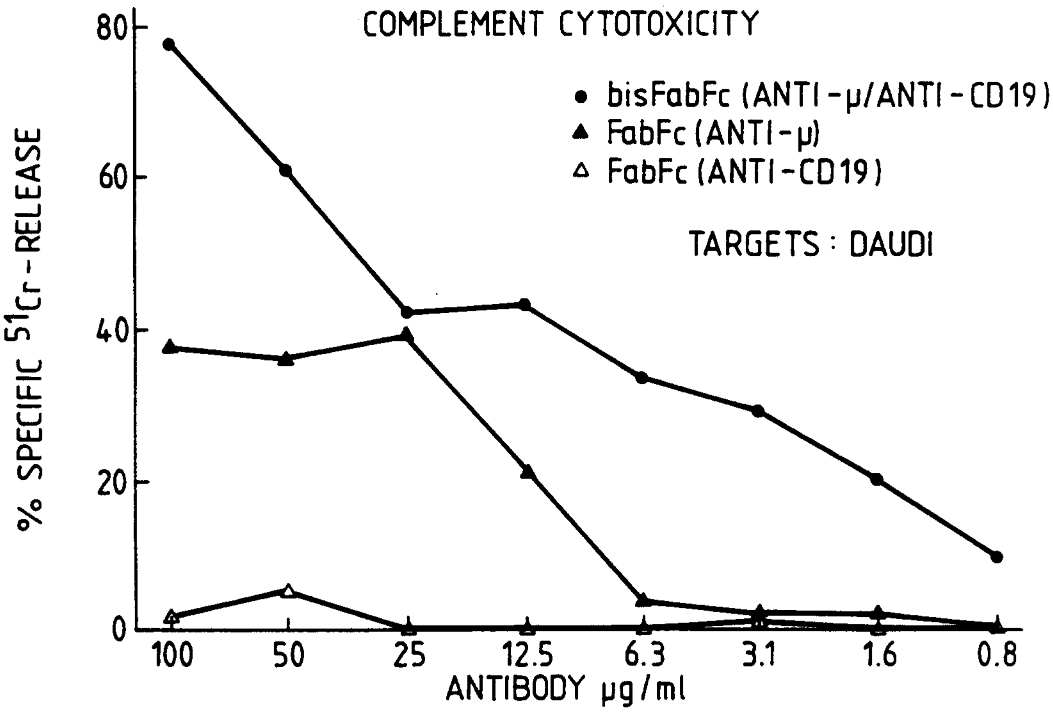
COMPLEMENT CYTOTOXICITY
● bisFabFc (ANTI-μ/ANTI-CD19)
▲ FabFc (ANTI-μ)
△ FabFc (ANTI-CD19)
TARGETS: DAUDI
% SPECIFIC 51Cr-RELEASE
80
60
40
20
0
100
50
25
12.5
6.3
3.1
1.6
0.8
ANTIBODY μg/ml

Fig.4B.

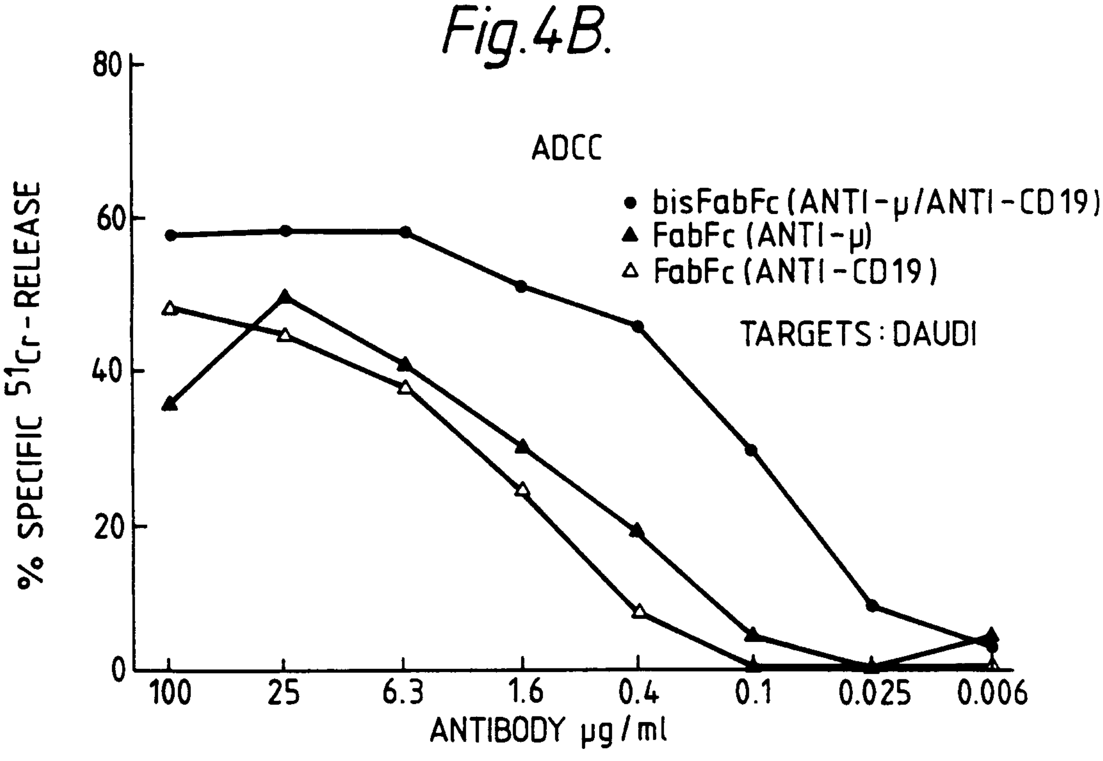
ADCC
● bisFabFc (ANTI-μ/ANTI-CD19)
▲ FabFc (ANTI-μ)
△ FabFc (ANTI-CD19)
TARGETS: DAUDI
% SPECIFIC 51Cr-RELEASE
80
60
40
20
0
100
25
6.3
1.6
0.4
0.1
0.025
0.006
ANTIBODY μg/ml

*Fig.5.*

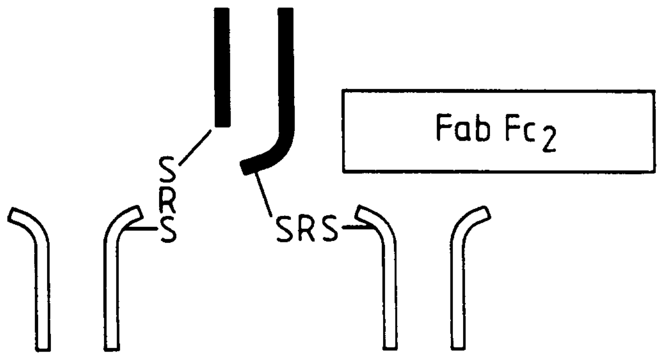


*Fig.6A.*

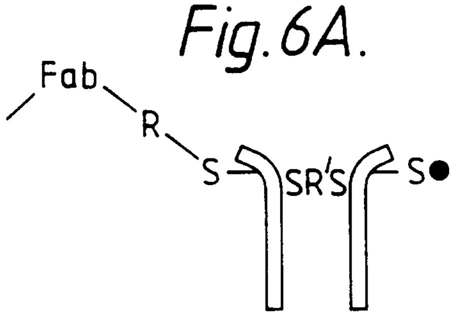


Homologous linkage

*Fig.6B.*

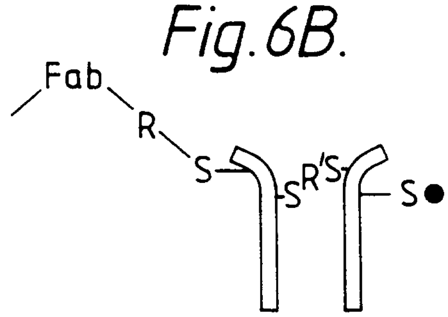


Non-homologous linkage

*Fig.6C.*

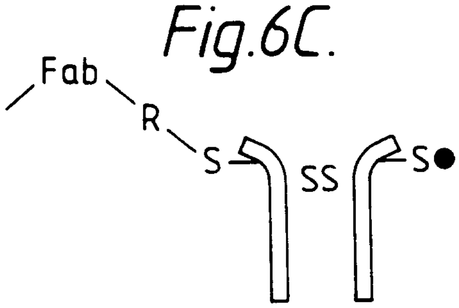


Closed-hinge Fc

*Fig.6D.*

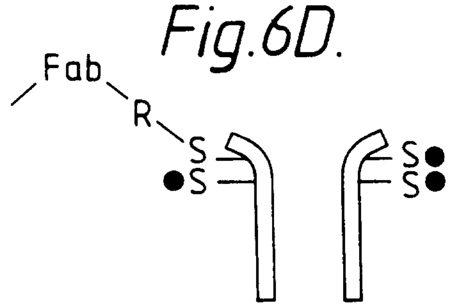


Open-hinge Fc

Fig. 7.

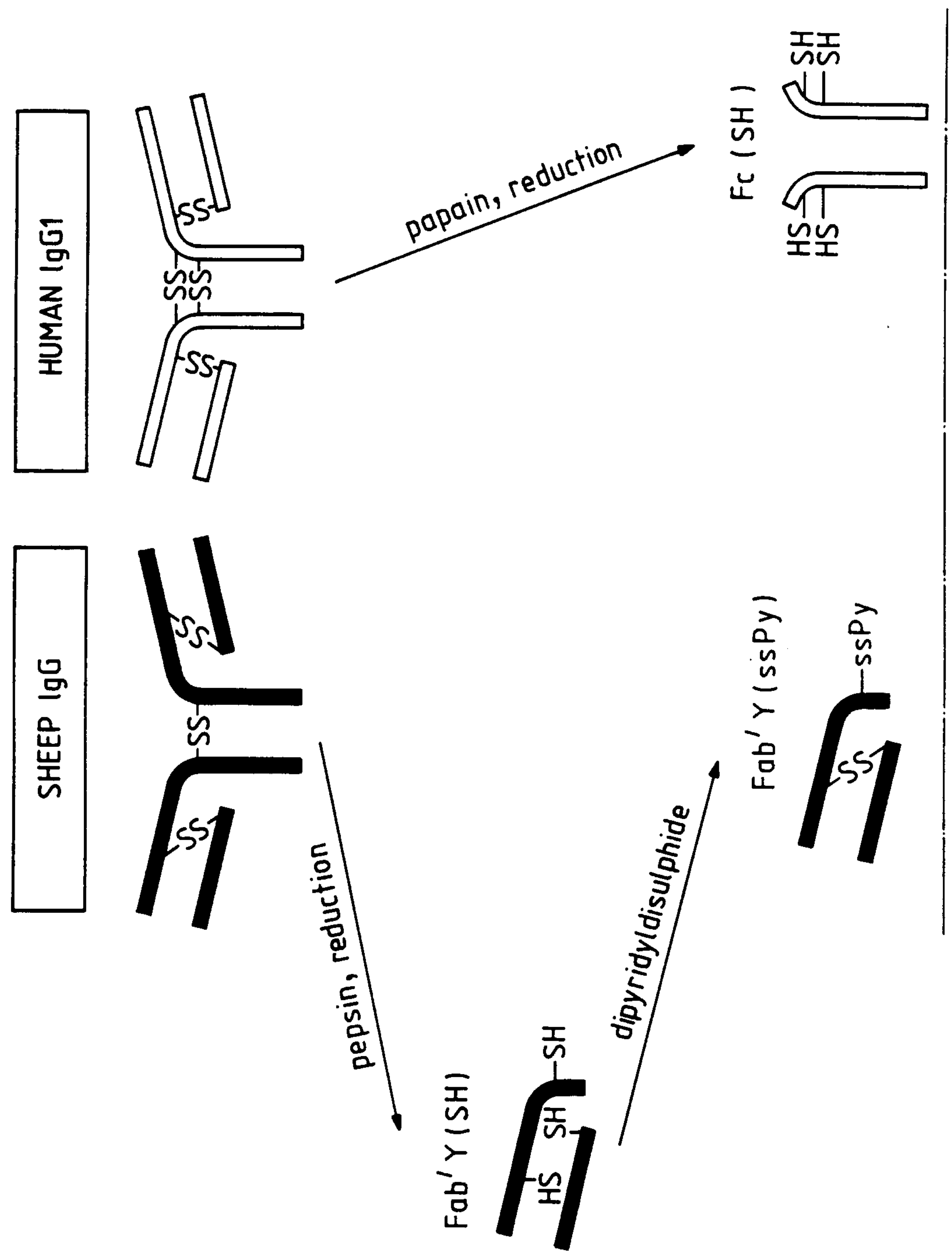
SHEEP IgG
HUMAN IgG1
SS
papain, reduction
Fc (SH)
HS
SH
pepsin, reduction
Fab' Y (SH)
dipyridyldisulphide
Fab' Y (ssPy)
ssPy

Fig.7(cont.)

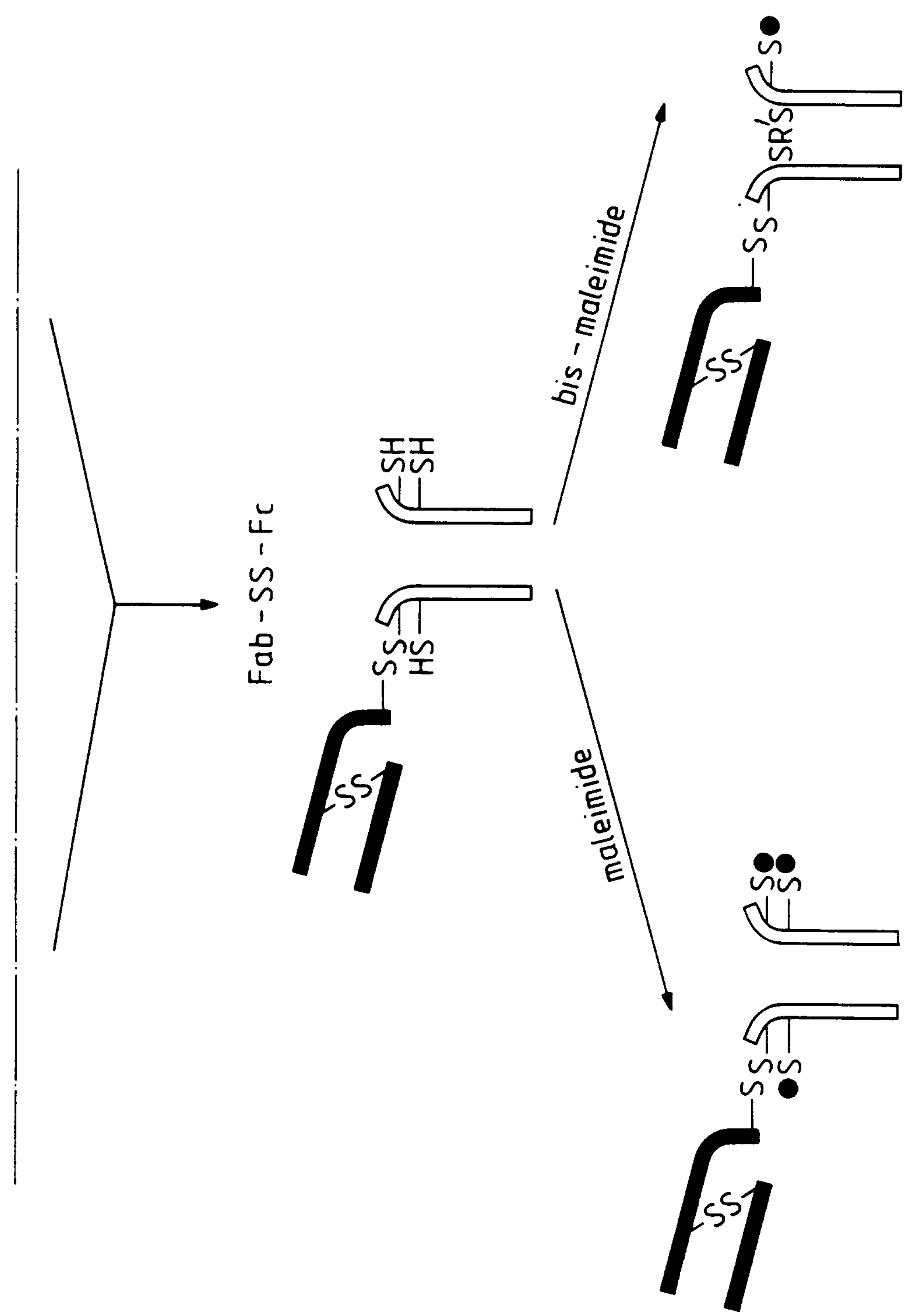

Fig.8.

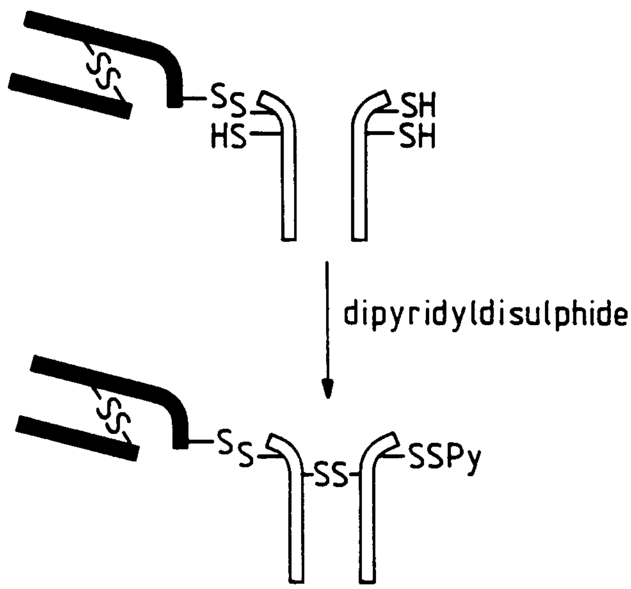
SS
HS
SH
SH
dipyridyldisulphide
SS
SS
SSPy

Fig.10.

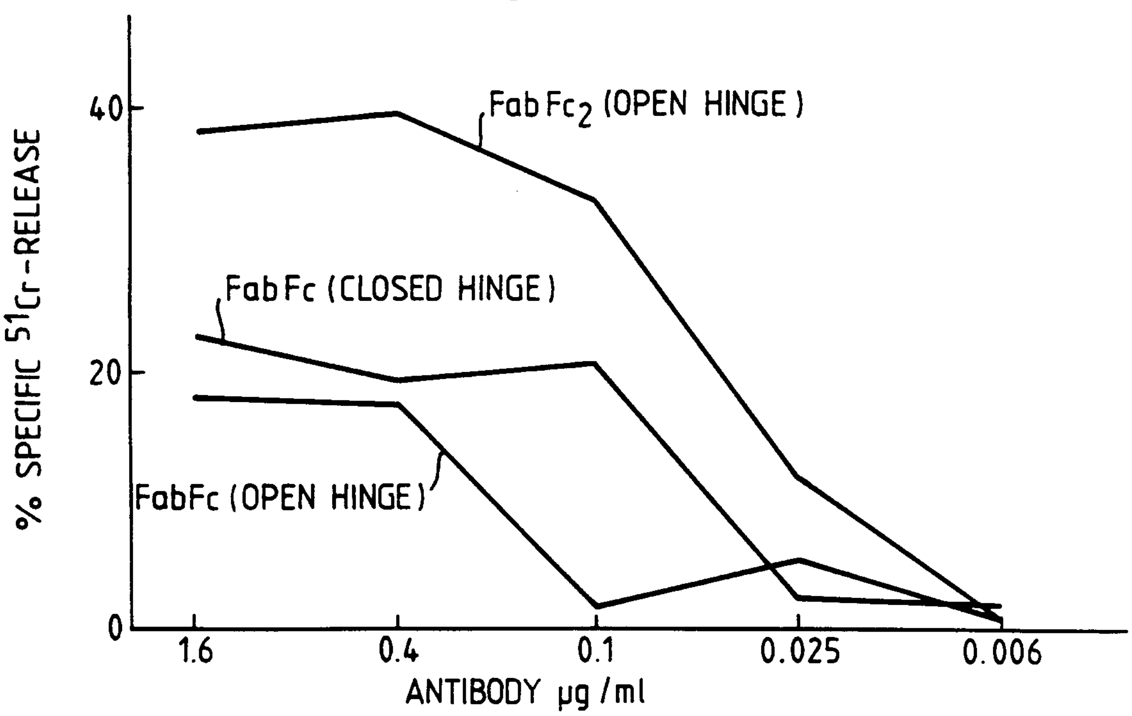
% SPECIFIC $^{51}$Cr-RELEASE
40
20
0
FabFc$_2$ (OPEN HINGE)
FabFc (CLOSED HINGE)
FabFc (OPEN HINGE)
1.6
0.4
0.1
0.025
0.006
ANTIBODY µg/ml

*Fig. 9.*

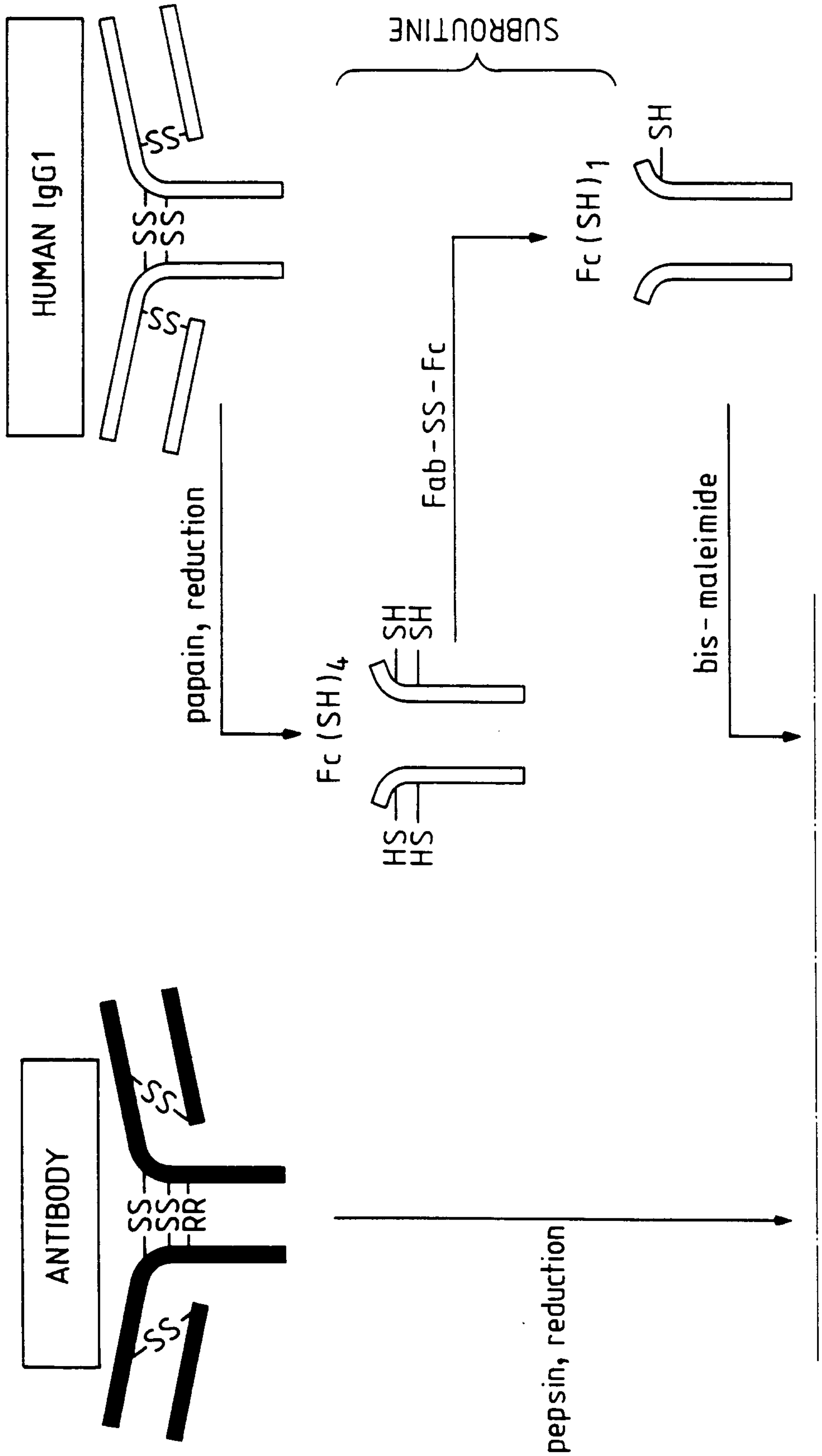
HUMAN IgG1
SS
SS
SS
SS
papain, reduction
Fc (SH)$_4$
HS
HS
SH
SH
Fab-SS-Fc
SUBROUTINE
Fc (SH)$_1$
SH
bis-maleimide
ANTIBODY
SS
SS
SS
RR
SS
pepsin, reduction

Fig.9(cont.)